(19) Europäisches Patentamt
European Patent Office
Office européen des brevets

(11) EP 0 640 606 B1

# (12) EUROPEAN PATENT SPECIFICATION

(45) Date of publication and mention of the grant of the patent:
**07.11.2001 Bulletin 2001/45**

(51) Int Cl.[7]: **C07D 495/04**, A61K 31/505, A61K 31/38

(21) Application number: **94113169.0**

(22) Date of filing: **24.08.1994**

## (54) Thienopyrimidine derivatives, their production and use

Thienopyrimidinderivate, ihre Herstellung und Verwendung

Dérivés de thiéropyrimidine, leur préparation et utilisation

(84) Designated Contracting States:
**AT BE CH DE DK ES FR GB GR IE IT LI LU NL PT SE**

(30) Priority: **26.08.1993 JP 21197293**
**29.06.1994 JP 14812694**

(43) Date of publication of application:
**01.03.1995 Bulletin 1995/09**

(73) Proprietor: **TAKEDA CHEMICAL INDUSTRIES, LTD.**
**Chuo-ku, Osaka 541 (JP)**

(72) Inventors:
- **Furuya, Shuichi**
  **Ibaraki 305 (JP)**
- **Choh, Nobuo**
  **Ibaraki 305 (JP)**
- **Ohtaki, Tetsuya**
  **Ibaraki 305 (JP)**
- **Watanabe, Toshifumi**
  **Osaka 586 (JP)**

(74) Representative: **Caffin, Lee et al**
**Takeda Euro Patent Office**
**Takeda Europe Research & Development Centre Ltd.**
**Savannah House**
**11-12 Charles II Street**
**London SW1Y 4QU (GB)**

(56) References cited:
**EP-A- 0 404 525** **EP-A- 0 443 568**

- **CHEMICAL ABSTRACTS, vol. 114, no. 11, 18 March 1991, Columbus, Ohio, US; abstract no. 102035e, K. OGAWA ET AL. 'Preparations of thienopyrimidinediones as aldose reductase inhibitors.' page 756 ;column 1 ; & JP-A-02 225 485 (TAIHO PHARMACEUTICAL CO., LTD.) 7 September 1990**
- **JOURNAL OF MEDICINAL CHEMISTRY, vol.34, no.4, April 1991, WASHINGTON US pages 1492 - 1503 M.S. MALAMAS ET AL. 'Quinazolineacetic Acids and Related Analogues as Aldose Reductase Inhibitors'**
- **CHEMICAL ABSTRACTS, vol. 106, no. 25, 22 June 1987, Columbus, Ohio, US; abstract no. 213890j, R. BOEHM ET AL. 'Thieno compounds. 6. Preparation of (3,4-dihydro-4-oxothieno[2, 3-d]pyrimidin-3-yl)- and (1,2,3,4-tetrahyd ro-2,4-dioxothieno[2,3-d]pyrimidinyl)alkan ecarboxylic acid derivatives.' page 658 ;column 2 ; & PHARMAZIE, vol.41, no.9, 1986 page 661**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 640 606 B1

**Description**

FIELD OF THE INVENTION

The present invention relates to endothelin antagonists containing thieno[2,3-d]pyrimidine derivatives, to thieno[2,3-d]pyrimidine derivatives and to methods of manufacturing them.

BACKGROUND OF THE INVENTION

The participation of endothelin has been suggested in adult diseases which have been increasing in recent years, particularly in those which are caused by ischemia such as cerebral infarction, angina pectoris, myocardial infarction and renal insufficiency. Endothelin is a peptide comprising 21 amino acids produced by endothelial cells and endothelin-1, endothelin-2 from which endothelin-3 have been obtained. In this specification, a group comprising these endothelins will be referred to as "endothelin".

It has been reported that endothelin exhibits the most powerful and long-lasting vasoconstrictive action, hypertensive activity and potentiating action for contraction of heart muscles among the natural and synthetic substances which were known hitherto. Such actions of the peptide have been thought to be due to the endothelin receptors which are thought to be present in smooth muscle membranes of the blood vessel and others. With regard to the endothelin receptors, endothelin-A receptor and endothelin-B receptor have been known. Hereinafter, they will be referred to as "endothelin receptors".

Accordingly, compounds having affinity for endothelin receptors while showing endothelin antagonistic activity have prophylactic and therapeutic effects against diseases which are caused by ischemia such as cerebral infarction, angina pectoris, myocardial infarction, renal insufficiency and liver insufficiency and thus development of these compounds has been greatly expected. With respect to compounds having an endothelin antagonistic action prepared by means of synthesis, those which are disclosed in the EP-A-510526 and 526708 and the WO-A-93/08799 have been known. However, the degree of endothelin antagonistic action of these compounds has not been satisfactory. EP 0 404 525 relates to sulfur-containing fused pyrimidine derivatives, their production and use.

An object of the present invention is to offer endothelin antagonists containing thienopyrimidine derivatives exhibiting an excellent endothelin antago3nistic action, novel thienopyrimidine derivatives and methods of manufacturing them.

Under the above-mentioned circumstances, the present inventors have carried out extensive investigations in order to find novel compounds which can be used as endothelin antagonists and found that the thieno[2,3-d]pyrimidine derivatives exhibit an excellent endothelin antagonistic action. As a result of further investigations based upon such a finding, the present inventors have achieved the present invention.

SUMMARY OF THE INVENTION

Thus, the present invention provides a pharmaceutical composition for antagonizing endothelin containing thieno[2,3-d]pyrimidine derivatives. The present invention also provides novel thienopyrimidine derivatives and salts thereof. The present invention further provides methods for manufacturing thieno[2,3-d]pyrimidine derivatives.

More specifically, the present invention provides:

1. a compound of the formula:

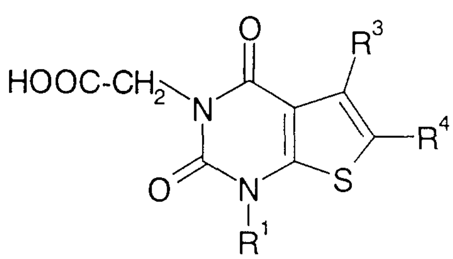

(I)

wherein

$R^1$ is benzyl which may be substituted by substituents selected from the group consisting of $C_{1-6}$ alkyl, $C_{1-6}$

alkoxy and $C_{1-6}$ alkylthio,
$R^3$ is hydrogen or $C_{1-6}$ alkyl,
$R^4$ is phenyl which may be substituted by $C_{1-6}$ alkoxy or $C_{1-6}$ alkylthio,

or a salt thereof,

2. a compound selected from the group consisting of

2,4(1H,3H)-dioxo-5-methyl-6-(4-methoxymethoxyphenyl)-1-(2-methoxybenzyl)thieno[2,3-d]pyrimidine-3-acetic acid,
2,4(1H,3H)-dioxo-5-methyl-6-(4-methoxyphenyl)-1-(2-methylthiobenzyl)thieno[2,3-d]pyrimidine-3-acetic acid,
2,4(1H,3H)-dioxo-5-methyl-6-(4-methoxymethoxyphenyl)-1-(2-methylthiobenzyl)thieno[2,3-d]pyrimidine-3-acetic acid,
2,4(1H,3H)-dioxo-5-methyl-6-(4-methylthiomethoxyphenyl)-1-(2-methylthiobenzyl)thieno[2,3-d]pyrimidine-3-acetic acid,
2,4(1H,3H)-dioxo-5-methyl-6-(4-methylthiomethoxyphenyl)-1-(2-methoxybenzyl)thieno[2,3-d]pyrimidine-3-acetic acid,
2,4(1H,3H)-dioxo-5-methyl-1-(2-methylthiobenzyl)-6-(4-propoxyphenyl)thieno[2,3-d]pyrimidine-3-acetic acid and
2,4(1H,3H)-dioxo-5-methyl-1-(2-methylthiobenzyl)-6-[4-(2-oxopropoxy)phenyl]thieno[2,3-d]pyrimidine-3-acetic acid;

3. a pharmaceutical composition comprising a compound of 1. or 2. and a pharmaceutically acceptable carrier, excipient or diluent;

4. use of a compound as defined in 1 for producing a pharmaceutical composition for antagonizing endothelin activity in a mammal suffering from an endothelin-derived disorder;

5. use according to 4, wherein the endothelin-derived disorder is selected from the group consisting of acute renal insufficiency, myocardial infarction and/or liver insufficiency;

6. use according to 5, wherein the endothelin-derived disorder is hypofunction of an organ caused by its surgery or transplant; and

7. use according to 6, wherein the organ is a liver.

## DETAILED DESCRIPTION OF THE INVENTION

The compounds of the present invention and the starting material compounds used for the production of them (hereinafter, both may be referred to as "the compounds of the present application" in some cases) will be illustrated in more detail.
In the above formula $C_{1-6}$ alkyl is exemplified by methyl, ethyl, propyl, isopropyl, butyl, tert-butyl, pentyl, and hexyl.
Examples of $C_{1-6}$ alkoxy are methoxy, ethoxy, butoxy, and hexyloxy.
Examples of $C_{1-6}$ alkylthio are methylthio, ethylthio, butylthio, and hexylthio.
Preferred examples of the compound (I) or salts thereof are, for example,

2,4(1H,3H)-dioxo-5-methyl-6-(4-methoxymethoxyphenyl)-1(2-methoxybenzyl)thieno[2,3-d]pyrimidine-3-acetic acid,
2,4(1H,3H)-dioxo-5-methyl-6-(4-methoxyphenyl)-1-(2-methylthiobenzyl)thieno [2,3-d]pyrimidine-3-acetic acid,
2,4(1H,3H)-dioxo-5-methyl-6-(4-methoxymethoxyphenyl)-1-(2-methylthiobenzyl)thieno[2,3-d]pyrimidine-3-acetic acid,
2,4(1H,3H)-dioxo-5-methyl-6-(4-methylthiomethoxyphenyl)-1-(2-methylthiobenzyl)thieno[2,3-d]pyrimidine-3-acetic acid,
2,4(1H,3H)-dioxo-5-methyl-6-(4-methylthiomethoxyphenyl)-1-(2-methoxybenzyl)thieno[2,3-d]pyrimidine-3-acetic acid,

2,4 (1H,3H)-dioxo-5-methyl-1-(2-methylthiobenzyl)-6-(4-propoxyphenyl)thieno[2,3-d]pyrimidine-3-acetic acid and 2,4(1H,3H)-dioxo-5-methyl-1-(2-methylthiobenzyl)-6-[4-(2-oxopropoxy)phenyl]thieno[2,3-d]pyrimidine-3-acetic acid or salts thereof.

In the following, description is made on the method of producing the compound (I), or a salt thereof.

(a) A compound (IV') of the formula:

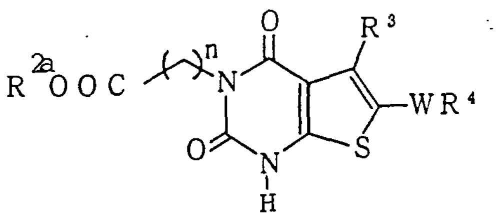

(IV')

wherein

| | |
|---|---|
| $R^{2a}$ | is an optionally-substituted hydrocarbon residue; |
| $R^3$ and $R^4$ | are as defined above; |
| W | is a chemical bond; and |
| n | is 1; |

or its salt is produced by subjecting a compound of the formula (II'):

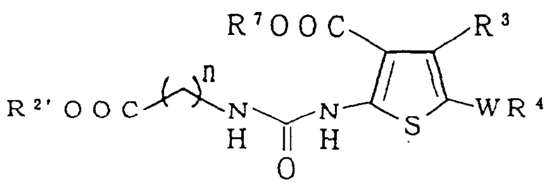

(II')

wherein

| | |
|---|---|
| $R^{2'}$ | is an optionally-substituted hydrocarbon residue; |
| $R^7$ | is hydrogen or an optionally-substituted hydrocarbon residue; and |
| $R^3$, $R^4$,W and n | are the same meanings as defined above, or its salt to a ring-closure reaction by using a base. |

The ring-closure reaction is carried out in a solvent which does not affect the reaction. Examples of the solvent are, for example, alcohol (e.g. methanol, ethanol, isopropanol) and ether (e.g. dioxane and tetrahydrofuran) etc.).

Examples of the base are, for example, alkali metal alkoxide (e.g. sodium methylate, sodium ethylate and sodium isopropoxide).

One molar portion of the compound (II') is employed with 1 to 5 moles, preferably 1.5 to 3 moles of the base.

The reaction temperature is about 10°C to boiling point of the employed solvent, preferably about: 25°C to boiling point of the employed solvent.

The reaction time is a few minutes to a few days, preferably 10 minutes to 2 days.

(b) A compound (IV') or its salt is produced by subjecting the compound (III) of the formula:

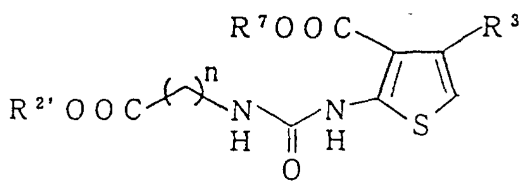

(III)

wherein each symbol is as defined above
or its salt to a ring-closure reaction by using a base and then subjecting the resulting ring-closure product to an electrophilic substitution reaction to introduce a group -$WR^4$ (in which W and $R^4$ are the same meaning as defined above).

The ring-closure reaction is carried out in a solvent which does not affect the reaction. Examples of the solvent are, for example, alcohol (e.g. methanol, ethanol and isopropanol), ether (e.g. dioxane and tetrahydrofuran).

Examples of the base are, for example, alkali metal alkoxide (e.g. sodium methylate, sodium ethylate and sodium isopropoxide).

One molar portion of the compound (II') is employed with 1 to 5 moles, preferably 1.5 to 3 moles of the base.

The reaction temperature is about 10°C to boiling point of the employed solvent, preferably about 25°C to boiling point of the employed solvent.

The reaction time is a few minutes to a few days, preferably 10 minutes to 2 days.

Examples of the electrophilic substitution reaction are per se known electrophilic substitution reaction. Preferred examples of the electrophilic substitution reaction are nitration (using, for example, fuming nitric acid-concentrated sulfuric acid and sodium nitrate-concentrated sulfuric acid), acylation (using, for example, an acid chloride-aluminum chloride), formylation (using, for example, phosphorus oxychloride-dimethylformamide and N-methylformanilide), halogenation (using, for example, N-bromosuccinimide, bromine-pyridine and sulfuryl chloride).

Said electrophilic substitution reaction can be carried out by a per se known method. For example, the nitration is carried out in fuming nitric acid-concentrated sulfuric acid or sodium nitrate-concentrated sulfuric acid at 0 to 80°C. For example, acylation is carried out by using alkanoylchloride (e.g. acetyl chloride and propionyl chloride) in a solvent, which does not affect the acylation, such as nitrobenzen, nitromethane and carbondisufide, in the presence of Lewis acid catalyst (e.g. aluminum chloride and titanium tetrachloride ) at

0 to 100°C. For example, the formylation is carried out by using phosphorous oxychloride-N,N-dimethylformamide or N-methylformanilide, oxalyl chloride-N,N-dimethylformamide or N-methylformanilide, thionyl chloride-N,N-dimethylformamide or N-methylformanilide in a solvent, which does not affect the formylation, such as benzene, toluene, xylene, tetrahydrofuran, dioxane, and 1,2-dichloroethane, at 15 to 130°C . For example, the halogenation is carried out by using sulfuryl chloride, N-chlorosuccinimide, N-bromosuccinimide, bromine, chlorine, iodine, in a solvent, which does not affect the halogenation, such as dichloromethane, chloroform, carbon tetrachloride, 1,2-dichloroethane, pyridine, benzene, toluene, and xylene, at 15 to 130°C .

If desired, the group which is introduced by the electrophlic substitution reaction can be subjected a functional group conversion reaction. Examples of the functional group conversion reaction are per se known functional group conversion reactions such as reduction, acylation, sulfonylation, alkylation, diazo-coupling, Wittig reaction, halogenation, Grignard reagent with halide, organic zinc reagent and organic boron reagent organic thin reagent.
(c) A compound (VI) of the formula:

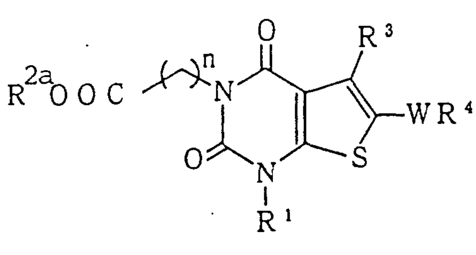

(VI)

wherein each symbol is as defined above, or its salt is produced by subjecting the compound (IV') or its salt, which is produced in above-mentioned (a) and (b), to react with a compound of the formula (V'):

$R^1$-X

wherein

$R^1$ is as defined above and
X is halogen,

or its salt.
Halogen shown by X is fluorine, chlorine, bromine or iodine.

The reaction is carried out in a solvent which does not affect the reaction. Examples of the solvent are ether (e.g. dioxane and tetrahydrofuran), aromatic hydrocarbon (e.g. benzene, toluene and xylene), amide (e.g. N,N-dimethylformamide, and N,N-dimethylacetamide) and dimethylsulfoxide, Preferably, the reaction is carried out in the presence of a base (e.g. potassium carbonate, sodium hydride, potassium hydride, and potassium t-butoxide).

One molar portion of the compound (IV') is employed with 1 to 5 moles, preferably 1.1 to 2.5 moles of the compound (V'). When a base is used in the reaction, one molar portion of the compound (IV') is employed with 1 to 5 moles, preferably 1.1 to 3 moles of the base.

The reaction temperature is 10°C to boiling point of the employed solvent, preferably 20°C to 130°C.

The reaction time is a few minutes to a few days, preferably 10 minutes to 2 days.

(d) A compound (I) or its salt is produced by subjecting the compound (VI) or its salt, which is produced in above-mentioned (c), to a reaction in which $R^{2a}$ is converted to hydrogen.

Example of the reaction, in which $R^{2a}$ is converted to hydrogen, is hydrolysis.

The hydrolysis is carried out by subjecting the compound (VI) or its salt to react a base in a solvent which does not affect the hydrolysis. Examples of the solvent are alcohol (e.g. methanol, ethanol and isopropanol), ether (e.g. dioxane and tetrahydrofuran), aromatic hydrocarbon (e.g. benzene, toluene and xylene), amide (e.g. N,N-dimethylformamide and N,N-dimethylacetamide), and dimethylsulfoxide. Examples of the base are alkali metal hydroxide (e.g. lithium hydroxide, sodium hydroxide, and potassium hydroxide), alkaline earth metal hydroxide (e.g. calcium hydroxide and barium hydroxide), alkali metal carbonate (e.g. potassium carbonate and sodium carbonate).

One molar portion of the compound (VI) is employed with 1 to 10 moles, preferably 1.5 to 5 moles of the base.

The reaction temperature is about 10°C to boiling point of the employed solvent, preferably about 15°C to 130°C.

The reaction time is a few minutes to a few days, preferably about 10 minutes to 2 days.

Production of a raw compound (II'), (III) or a salt thereof, used in the above-mentioned producing method (a) to (d), can be, for example, carried out by a method A or B as set forth below.

1. Method A.

The compound (II'), (III) or a salt thereof is produced by subjecting a compound of the formula (VIII):

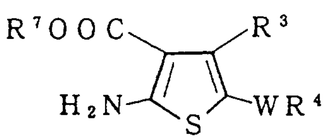

(VIII)

wherein $R^3$, $R^4$, $R^7$, W and n are the same meaning as defined above, or its slat; or a compound of the formula (VIII'):

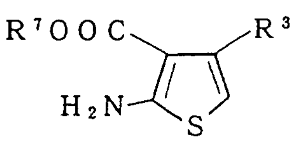

(VIII')

wherein $R^3$ and $R^7$ are the same meaning as defined above, or its salt to react with an isocyanate derivative.

Examples of the isocyanate derivative are an isocyanate ester derivative of the formula: $R^7OOC-(CH_2)n-NCO$ (in which $R^7$ and n are the same meaning as defined above.

The reaction is carried out in a solvent which does not affect the reaction. Examples of the solvent are tetrahydrofuran, pyridine, dioxane, benzene, dichloromethane, 1,2-dichloroethane, toluene and xylene).

One molar portion of the compound (VIII) or (VIII) is employed with . 1 to 5 moles, preferably 1.1 to 2.5 moles of the icocyanate derivative.

The reaction temperature is 15°C to 130°C, preferably 25°C to 130°C.

The reaction time is a few minutes to a few days, preferably 10 minutes to 2 days.

2. Method B.

The compound (II'), (III) or a salt thereof is produced by subjecting the compound (VIII), (VIII') or a salt thereof to react with phosgene or an equivalent thereof [e.g. diphosgene such as bis(trichloromethyl)carbonate, triphosgene such as trichloromethylchloroformate to give an isocyanate derivative, and then subjecting the isocyanate derivative to react with amine [e.g. a compound of the formula: $R^7OOC-(CH_2)n-NH_2$ (in which $R^7$ and n are the same meaning as defined above].

The reaction of the compound (VIII), (VIII') or its salt with the phosgene or its equivalent is carried out in a solvent which does not affect the reaction. Examples of the solvent are tetrahydrofuran, pyridine, dioxane, benzene, dichloromethane, 1,2-dichloroethane, toluene and xylene).

One molar portion of the compound (VIII) or (VIII') is employed with 0.5 to 2 moles, preferably 0.9 to 1.1 moles of the phosgene or its equivalent.

The reaction temperature is 15°C to 130°C, preferably 25°C to 130°C.

The reaction time is a few minutes to a few days, preferably 10 minutes to 2 days.

The reaction of the isocyanate derivative with the amine is carried out in a solvent which does not affect the reaction. Examples of the solvent are tetrahydrofuran, pyridine, dioxane, benzene, dichloromethane, 1,2-dichloroethane, toluene and xylene).

One molar portion of the compound (VIII) or (VIII') is employed with 1 to 5 moles, preferably 1.1 to 3 moles of the phosgene or its equivalent.

The reaction temperature is 15°C to 130°C, preferably 25°C to 130°C .

The reaction time is a few minutes to a few days, preferably 10 minutes to 2 days.

The compound (VIII) or its salt used in the above reaction is produced by the reaction of a ketone or an aldehyde having an activated methylene group [e.g. a compound of the formula (IX): $R^3-CO-CH_2-WR^4$ (in which $R^3$, $R^4$ and W are the same meanings as defined above)] with a cyanoacetate derivative and sulfur according to a method by Karl Gewald, et al. [K. Gewald, E. Schinke and H. Boettcher: Chem. Ber., 99, 94-100 (1966)]. Thus, in the case of a ketone [e.g. a compound (IX) in which $R^3$ is a group bonded through a nitrogen atom or a carbon atom], it is made to react with a cyanoacetate derivative in the presence of acetic acid and ammonium acetate are refluxed in a solvent, which does not affect the reaction, such as benzene, toluene and the resulting alkylidene cyanoacetate derivative is heated (e.g. 50 to 80°C) in the presence of sulfur and a base (e.g. an organic base such as triethylamine, ethyldiisopropyoamine, and dimethylaminopyridine) in a solvent, which does not affect the reaction, such as methanol, ethanol to give a 2-aminothiophene derivative [the compound (VIII) in which $R^3$ is $C_{1-6}$ alkyl.

In the case of an aldehyde [e.g. a compound (IX) in which $R^3$ is a hydrogen atom], it is heated with a cyanoester derivative in the presence of sulfur and a base (e.g. an organic base such as triethylamine, and ethyldiisopropylamine), in a solvent, which does not affect the reaction, such as dimethylformamide, dimethylsulfoxide to give a 2-aminothiophene derivative [a compound (VIII) in which $R^3$ is a hydrogen atom].

The compound (VIII') can be synthesized by a method of Karl Gewald, et al. [K. Gewald: Chem. Ber. 98, 3571-3577(1965); and K. Gewald and E. Schinke: Chem. Ber. 99, 2712-2715(1966)].

Examples of the hydrocarbon residue in the optionally-substituted hydrocarbon residue shown by the above-mentioned $R^{2a}$, $R^{2'}$ and $R^7$ are alkyl (e.g. $C_{1-6}$ alkyl such as methyl, ethyl, propyl, isopropyl, butyl, tert-butyl, pentyl and hexyl), cycloalkyl (e.g. $C_{3-6}$ cycloalkyl such as cyclopropyl, cyclopentyl and cyclohexyl), alkoxyalkyl (e.g. $C_{1-3}$ alkoxy $C_{1-6}$ alkyl such as methoxymethyl, ethoxymethyl, ethoxybutyl, propoxymethyl, and propoxyhexyl) hydroxyalkyl (e.g. hydroxy $C_{1-6}$ alkyl such as hydroxymethyl, hydroxyethyl, hydroxybutyl and hydroxypropyl), alkenyl (e.g. $C_{2-6}$ alkenyl such as vinyl, butadienyl and hexatrienyl), formyl, carboxyl, alkoxycarbonyl (e.g. $C_{1-6}$ alkoxycarbonyl such as methoxycarbonyl, ethoxycarbonyl and tert-butoxycarbonyl), aryl (e.g. $C_{6-14}$ aryl such as phenyl, naphthyl, and anthracenyl), aralkyl (e.g. $C_{7-20}$ aralkyl such as benzyl, benzhydryl and trityl).

Examples of the substituent that said hydrocarbon residue may have are nitro, hydroxyl, oxo, thioxo, cyano, carbamoyl, carboxyl, $C_{1-4}$ alkoxy-carbonyl (e.g. methoxycarbonyl and ethoxycarbonyl), sulfo, halogen (e.g. fluorine, chlorine, bromine and iodine), $C_{1-6}$ alkoxy (e.g. methoxy, ethoxy, propoxy, isopropoxy, butoxy, isobutoxy, sec-butoxy

and tert-butoxy), $C_{6-12}$ aryloxy (e.g. phenoxy), halogeno $C_{6-16}$ aryl (e.g. o-, m- or p-chlorophenoxy and o-, m- or p-bromophenoxy), $C_{1-6}$ alkylthio (e.g. methylthio, ethylthio, n-propylthio, isopropylthio, n-butylthio and tert-butylthio), $C_{6-12}$ arylthio (e.g. phenylthio), $C_{1-6}$ alkylsulfinyl (e.g. methylsulfinyl and ethylsulfinyl), $C_{1-6}$ alkylsulfonyl (e.g. methylsulfonyly and ethylsulfonyl), amino, $C_{1-6}$ acylamino (e.g. formylamino, acetylamino, and propionylamino), mono- or di-$C_{1-4}$ alkylamino (e.g. methylamino, ethylamino, n-propylamino, isopropylamino, n-butylamino, dimethylamino and diethylamino), $C_{1-6}$ acyl (e.g. formyl, acetyl and hexanoyl), $C_{6-12}$ aryl-carbonyl (e.g. benzoyl), a five- or six-membered heterocyclic group having one to four heteroatom(s) selected from oxygen, sulfur, nitrogen, etc. besides carbon atoms (e.g. 2- or 3-thienyl, 2- or 3-furyl, 3-, 4- or 5-pyrazolyl, 2-, 4- or 5-thiazolyl, 3-, 4- or 5-isothiazolyl, 2-, 4- or 5-oxazolyl, 3-, 4- or 5-isoxazolyl, 2-, 4-or 5-imidazolyl, 1,2,3- or 1,2,4-triazolyl, 1H- or 2H-tetrazolyl, 2-, 3- or 4-pyridyl, 2-, 4- or 5-pyrimidyl, 3- or 4-pyridanidyl, quinolyl, isoquinolyl and indolyl), which may be susbtituted with one to four substituent (s) selected from (a) halogen (e.g. fluorine, bromine, chlorine and iodine), (b) $C_{1-4}$ alkyl (e.g. methyl, ethyl, propyl and isopropyl) and (c) halogehophenoxy (e.g. o-, m- or p-chlorophenoxy, o-, m- or p-bromophenoxy), $C_{1-10}$ haloalkyl (e.g. difluoromethyl, trifluoromethyl, trifluoroethyl and trichloroethyl). One to five substituent(s) selected from the above may be used. When the hydrocarbonyl group is cycloalkyl, cycloalkenyl, aryl or aralkyl, the substituent may be $C_{1-6}$ alkyl (e.g. methyl, ethyl, propyl, isopropyl and butyl). Number of the substituent may be from noe to six or, preferably, one to three.

With respect to a salt of the compound of the present invention prepared as such, a physiologically acceptable acid addition salt is preferred. Examples of such a salt are the salts with inorganic acids (e.g. hydrochloric acid, hydrobromic acid, nitric acid, sulfuric acid and phosphoric acid) and the salts with organic acids (e.g. formic acid, acetic acid, trifluoroacetic acid, fumaric acid, oxalic acid, tartaric acid, maleic acid, citric acid, succinic acid, malic acid, methanesulfonic acid, benzenesulfonic acid and p-toluenesulfonic acid). Further, when the compound (I) of the present invention has an acidic group such as -COOH, the compound (I) may form a salt with an inorganic base (e.g. an alkali metal or an alkali earth metal such as sodium, potassium, calcium and magnesium; and ammonia) or an organic base (e.g. trimethylamine, triethylamine, pyridine, picoline, ethanolamine, diethanolamine, triethanolamine, dicyclohexylamine and N,N'-dibenzylethylenediamine).

The compound or a salt thereof prepared as such can be isolated and purified by conventional separating means such as recrystallization, distillation and chromatography. When the compound (I) is obtained in a free state, it can be converted to a salt by a known method per se or by a similar method thereto while, when it is obtained in a form of a salt, it can be converted to a free state or to another salt.

The salt of the above-mentioned compounds may be the same as that of the compound (I).

When the compound of the present invention or a salt thereof is an optically active substance, it can be separated into a dand an 1-compounds by a conventional means for optical resolution.

The compound of the present invention has an endothelin antagonistic activity with low toxicity thus rendering such compounds therapeuticall and diagnostically useful. Accordingly, it can be safely used as an endothelin antagonist to warm-blooded mammals (e.g. rats, mice, rabbits, cats, dogs, cattle, horses, and human being) especially for treating or preventing acute renal insufficiency, myocardial infarction, liver insufficiency, angina pectoris, cerebral infarction, sub-arachnoid haemorrhage (SAH), hypertension, renal insufficiency, asthma, variant form of angina, Raynaud's syndrome, pulmonary hypertension, surgery shock, chronic cardiac insufficiency, cardiac hypertrophy, arteriosclerosis and migraine, furthermore, for treating or preventing a hypofunction of an organ (e.g. liver) caused by its surgery or transplant, insufficient microcirculation, still fruthermore, for preventing restenosis after percutaneous transluminal coronary angioplasty(PTCA). More especially, it can be used for treating or preventing acute renal insufficiency, myocardial infraction, liver insufficiency, hypertension, pulmonary hypertension; for treating or preventing a hypofunction of an organ (e.g. liver) caused by its surgery or transplant, insufficient microcirculation; and for preventing restenosis after PTCA.

When the compound (I) or its salt is administered, for example, to human being, it can be safely administered either orally or parenterally as it is or as a pharmaceutical composition prepared by mixing with suitable pharmaceutically acceptable carriers, diluents and excipient.

Examples of the above-mentioned pharmaceutical composition are oral agents (e.g. diluted powders, granules, capsules and tablets), injections, dropping injections, external agents (e.g. transnasal preparations and percutaneous preparations), ointments (e.g. rectal ointment and vaginal ointment).

Such pharmaceutical compositions can be manufactured by a per se known method commonly used in preparing pharmaceutical compositions.

The compound (I) of the present invention or a salt thereof can be made into injections either in a form of an aqueous injection together with dispersing agents [e.g. Tween 80® (Atlas Powder, U. S. A.), HCO 80® (Nikko Chemicals, Japan), polyethylene glycol, carboxymethylcellulose and sodium alginate ], preservatives (e.g. methyl paraben, propyl paraben, benzyl and alcohol), isotonizing agents (e.g. sodium chloride, mannitol, sorbitol and glucose) or in a form of an oily injection by dissolving, suspending or emulsifying in plant oil (e.g. olive oil, sesame oil, cotton seed oil and corn oil), and propylene glycol.

In preparing a pharmaceutical composition for oral use, the compound (I) of the present invention or a salt thereof is molded by compressing, for example, with fillers (e.g. lactose, sucrose, and starch) disintegrating agents (e.g. starch, calcium and carbonate) binders (e.g. starch, gum arabic, carboxymethylcellulose, polyvinylpyrrolidone, and hydroxypropylcellulose) or lubricants (e.g. talc, magnesium stearate and polyethylene glycol 6000)® . If necessary, the composition is coated by a per se known method with an object of masking the taste, enteric caoting or long-acting. Examples of the coating agent therefor are hydroxypropylmethylcellulose, ethylcellulose, hydroxymethylcellulose, hydroxypropylcellulose, polyoxyethylene glycol, Tween 80® , pluronic F 68® , cellulose acetate phthalate, hydroxypropylmethylcellulose phthalate, hydroxymethylcellulose acetate succinate, Eudragit® (a copolymer of methacrylic acid with acrylic acid; manufactured by Rohm, Germany), red oxide of iron.

Subcoating layer may be provided between the enteric coating and the core according to per se known method.

In preparing an external composition, the compound (I) of the present invention or a salt thereof as it is or a salt thereof is subjected to a per se known method to give a solid, semisolid or liquid agent for external use. For example, the solid preparation is manufactured as follows. Thus, the compound (I) as it is or after adding/mixing fillers (e.g. glycol, mannitol, starch, and microcrystalline cellulose), thickeners (e.g. natural gums, cellulose derivatives and acrylic acid polymers), thereto/therewith is made into a powdery composition. With respect to the liquid composition, an oily or aqueous suspension is manufactured by the manner nearly the same as in the case of the injection. In the case of a semisolid composition, the preferred one is an aqueous or oily gel or an ointment. Each of them may be compounded with a pH adjusting agent (e.g. carbonic acid, phosphoric acid, citric acid, hydrochloric acid, sodium and hydroxide) and an antiseptic agent (e.g. p-hydroxybenzoates, chlorobutanol and benzalkonium chloride).

In the manufacture of an ointment for example, the compound (I) of the present invention or a salt thereof can be made into an oily or an aqueous solid, semisolid or liquid ointment. Examples of the oily base material applicable in the above-mentioned composition are glycerides of higher fatty acids [e.g. cacao butter, Witepsols® (manufactured by Dynamite-Nobel), etc.], medium fatty acids [e.g. Miglyols® (manufactured by Dynamite-Nobel) ] and plant oil (e.g. sesame oil, soybean oil and cotton seed oil).

Examples of the aqueous base material are polyethylene glycols and propylene glycol and those of the base material for aqueous gel are natural gums, cellulose derivatives, vinyl polymers and acrylic acid polymers.

A daily dose may vary depending upon the degree of the disease; age, sex, body weight, a difference in the susceptibility, of the patient; time and interval of the administration; nature, composition and type of the pharmaceutical composition; type of the active component; and is not particularly limited. Usually, however, it is 0.01-150 mg/kg, preferably 0.1-100 mg/kg or, more preferably, 0.5-50 mg/kg to warm-blooded animals. The above dose is usually administered by dividing it into one to four times a day.

(Examples)

The present invention will be further illustrated by way of the following examples though the present invention is not to be limited thereto.

$^1$H-NMR spectrum is determined by a Varian Gemini 200 (200 MHz) type spectrometer or Bruker AM-500 (500 MHz) type spectrometer using tetramethyl silane as the internal standard, expressing all the values as ppm.

Symbols used in the reference examples and working examples are of the following meaning.

s; singlet, d; doublet, t; triplet, q; quartet, dd; double doublet; dt; double triplet, m; multiplet, br; broad, J; coupling constant.

The following compounds are within the scope of the present invention:
Compound 92 of Example 22 and Compounds 99, 100, 102, 105, 110, 111, 115, 119, 122-125, 128, 129, 131, 133, 140, 144-146, 148, 151, 157 and 158 of Examples 23. All the other Reference Examples and Examples and Compounds are illustrative for the production of intermediates and for the production of the claimed compounds.

Reference Example 1.

Production of ethyl 2-amino-5-phenylthiophene-3-carboxylate.

Phenylacetaldehyde (a 50% solution in diethyl phthalate;
12.05 g; 50 mmoles) was added dropwise, within 20 minutes, into a mixture of ethyl cyanoacetate (6.1 g, 50 mmoles), sulfur (1.61 g, 50 mmoles), triethylamine (3.5 ml, 25 mmoles) and dimethylformamide (10 ml). The mixture was stirred at 45°C for nine hours. The reaction mixture was concentrated and the resulting residue was extracted with ethyl acetate. The extract was washed with an aqueous solution of sodium chloride, then dried over $MgSO_4$ and evaporated in vacuo. The resulting residue was chromatographed on silica gel followed by crystallizing from ether-hexane to give pale yellow plates (5.55 g; 45%).

m.p. 124.5-125.5°C (the literature value: 123-124°C).

| Elemental analysis (%) for $C_{13}H_{13}NO_2S$: | | | |
|---|---|---|---|
| Calcd. | C 63.13, | H 5.30, | N 5.66; |
| Found | C 62.99, | H 5.05, | N 5.63. |

$^1$H-NMR (200 MHz, $CDCl_3$) δ : 1.37 (3H, t, J = 7.1Hz), 4.30 (2H, d, J = 7.1Hz), 5.97 (2H, br), 7.17-7.46 (6H m)
IR (KBr): 3448, 3320, 1667, 1590, 1549 $cm^{-1}$.

Reference Example 2.

Production of ethyl 2-amino-4-methyl-5-(4-methoxyphenyl)-thiophene-3-carboxylate.

A mixture of 4-methoxyphenylacetone (16.5 g, 0.10 mole), ethyl cyanoacetate (12.2 g, 0.10 mole), ammonium acetate (1.55 g, 20 mmoles), acetic acid (4.6 ml, 80 mmoles) and benzene (20 ml) was refluxed for 24 hours together with a removal of the resulting water therefrom by means of a Dene-Starke's apparatus. After cooling, the reaction mixture was concentrated in vacuo and the resulting residue was partitioned between dichloromethane and an aqueous solution of sodium bicarbonate. The organic layer was washed with an aqueous solution of sodium chloride, then dried over $MgSO_4$ and evaporated in vacuo. The resulting residue was taken up in ethanol (30 ml). To the solution were added sulfur (3.21 g, 0.10 mole) and diethylamine (10.4 ml, 0.10 mole), and the mixture was stirred at 50-60°C for two hours. The reaction mixture was concentrated and the resulting residue was extracted with ethyl acetate. The extract was washed with an aqueous solution of sodium chloride, then dried over $MgSO_4$ and evaporated in vacuo. The resulting residue was chromatographed on silica gel followed by crystallizing from ether-hexane to give pale yellow plates (11.5 g; 40%).

m.p. 79-80°C .

| Elemental analysis (%) for $C_{15}H_{17}NO_3S$: | | | | |
|---|---|---|---|---|
| Calcd. | C 61.83, | H 5.88, | N 4.81, | S 11.01; |
| Found | C 61.81, | H 5.75, | N 4.74, | S 10.82. |

$^1$H-NMR (200MHz, $CDCl_3$) δ : 1.37 (3H, t, J = 7.1Hz), 2.28 (3H, s), 3.83 (3H, s), 4.31 (2H, q, J = 7.1Hz), 6.05 (2H, brs), 6.91 (2H, d, J = 8.8Hz), 7.27 (2H, d, J = 8.8Hz).
IR (KBr): 3426, 3328, 1651, 1586, 1550, 1505, 1485 $cm^{-1}$
FAB-MS m/z: 291 ($M^+$).

Reference Example 3.

Production of ethyl 2-amino-4-methyl-5-phenylthiophene-3-carboxylate.

Phenylacetone (11.6 g; 86.5mmoles) was used instead of 4-phenylacetone and the same operations as mentioned in Reference Example 2 were carried out using ethyl cyanoacetate (10.5 g, 86.5 mmoles), ammonium acetate (1.34 g, 17.4 mmoles), acetic acid (3.96 ml, 69.2 mmoles), sulfur (2.78 g, 86.5 mmoles) and dietylamine (8.95 ml, 86.5 mmoles) to give colorless needles (9.95 g; 40%).

m.p. 64-65°C (recrystallized from ether-hexane; lit: 95°C).

| Elemental analysis (%) for $C_{14}H_{15}NO_2S$: | | | |
|---|---|---|---|
| Calcd. | C 64.34, | H 5.79, | N 5.36; |
| Found | C 64.51, | H 5.77, | N 5.29. |

$^1$H-NMR (200MHz, $CDCl_3$) δ : 1.37 (3H, t, J = 7.1Hz), 2.33 (3H, s), 4.32 (2H, q, J = 7.1Hz), 6.09 (2H, br), 7.24-7.42 (5H, m).
IR (KBr): 3388, 3278, 1665, 1584, 1549, 1481 $cm^{-1}$.

Reference Example 4.

Production of ethyl 2-amino-4-methyl-5-(3,4-methylenedioxyphenyl)thiophene-3-carboxylate.

3,4-Methylenedioxyphenylacetone (5.34 g; 30 mmoles) was used instead of 4-phenylacetone and the same operations as in Reference Example 2 were carried out by using ethyl cyanoacetate (3.65 g, 30 mmoles), ammonium acetate (0.46 g, 6 mmoles), acetic acid (1.37 ml, 24 mmoles), sulfur (0.90 g, 28.25 mmoles) and diethylamine (2.92 ml, 28.25 mmoles) to give pale yellow powders (2.90 g; 34%).

$^1$H-NMR (200MHz, $CDCl_3$) δ : 1.37 (3H, t, J = 7.1Hz), 2.29 (3H, s), 4.31 (2H, q, J = 7.1Hz), 5.99 (2H, s), 6.82 (3H, s).

Reference Example 5.

Production of ethyl 2-amino-4-methyl-5-(3,4-dimethoxyphenyl)thiophene-3-carboxylate.

3,4-Dimethoxyphenylacetone (5.0 ml; 28.7 mmoles) was used instead of 4-phenylacetone and the same operations as in Reference Example 2 were carried out by using ethyl cyanoacetate (0.44 g, 5.74 mmoles), ammonium acetate (3.49 g, 28.7 mmoles), acetic acid (1.32 ml, 23.0mmoles), sulfur (0.90 g, 28.0 mmoles) and diethylamine (2.9 ml, 28.0 mmoles) to give pale yellow powders (3.09 g; 34%).

$^1$H-NMR (200 MHz, $CDCl_3$) δ : 1.38 (3H, t, J = 7.1Hz), 2.30 (3H, s), 3.89 (3H, s), 3.91 (3H, s), 4.32 (2H, q, J = 7.1 Hz), 6.07 (2H, s), 6.74-6.89 (3H, m).

Reference Example 6.

Production of ethyl 2,4(1H,3H)-dioxo-5-methylthioeno[2,3-d]pyrimidine-3-acetate.

Ethyl isocyanate acetate (9.1 ml; 81.1 mmoles) was added dropwise into a solution of ethyl 2-amino-4-methylthiophene (10.0 g, 54.0 mmoles) in pyridine (25 ml) at 45-50°C. The mixture was stirred for two hours. The reaction mixture was evaporated to dryness and the resulting residue was partitioned between ethyl acetate and diluted hydrochloric acid. The aqueous layer was extracted with ethyl acetate. The combined extracts were washed with an aqueous solution of sodium chloride, dried over $MgSO_4$ and evaporated in vacuo. The resulting residue was recrystallized from ethyl acetate to give colorless needles (16.1 g). The crystals (12.0 g; 38.2 mmoles) were suspended in ethanol (150 ml), then sodium ethoxide [prepared from metal sodium (1.95 g, 84.8 mmoles) and ethanol (70 ml)] was added thereto and the mixture was stirred at room temperature for one hour. To the reaction mixture was added 2N hydrochloric acid (45 ml) under ice-cooling and ethanol was evaporated therefrom in vacuo. The crystals separated out therefrom were collected by filteration, washed with water ethanol, dried over phosphorus pentaoxide in vacuo and recrystallized from ethanol to give colorless needles (9.50 g; 93%).

m.p. 229-230°C .

Reference Example 7.

Production of ethyl 2,4(1H,3H)-dioxo-4-nitro-5-methylthieno[2,3-d]pyrimidine-3-acetate.

To the compound produced in Reference Example 6 (4.0 g, 14.9 mmoles) was added concentrated sulfuric acid (15 ml) and then a solution of sodium nitrate (1.33 g, 15.7 mmoles) in concentrated sulfuric acid (25 ml) was added dropwise thereinto with ice-cooling over 15 minutes. After the addition was complete, the mixture was stirred under ice-cooling for one hour. The reaction mixture was poured into ice-water and extracted with a mixture of ethyl acetate and tetrahydrofuran. The extract was washed with an aqueous solution of sodium chloride, dried over $MgSO_4$ and evaporated in vacuo. The resulting residue was chromatographed on silica gel to give a white solid (4.08 g; 86%). The solid was recrystallized from ethyl acetate-hexane to give yellow needles.

m.p. 214-215°C .

| Elemental analysis (%) for $C_{11}H_{11}N_3O_6S \cdot 0.1H_2O$: | | | |
|---|---|---|---|
| Calcd. | C 41.93, | H 3.58, | N 13.34; |
| Found | C 41.90, | H 3.88, | N 13.24. |

$^1$H-NMR (200 MHz, DMSO-$d_6$) δ : 1.35 (3H, t, J = 7.1Hz), 2.95 (3H, s), 4.30 (2H, q, J = 7.1Hz), 4.75 (2H, s), 10.66 (1H, brs).

IR (KBr): 3530, 1760, 1719, 1676, 1549, 1444, 1321 $cm^{-1}$.

Reference Example 8.

Production of ethyl 2,4(1H,3H)-dioxo-1-(2-methoxybenzyl)-5-methyl-6-nitrothieno[2,3-d]pyrimidine-3-acetate.

A solution of the compound produced in Reference Example 7 (0.912 g, 5.87 mmoles) in dimethylformamide (6 ml) was added dropwise into a suspension of sodium hydride (0.125 g, 3.13 mmoles, which was washed with n-Hexane) in dimethylformamide (2 ml) under nitrogen stream with ice-cooling. The mixture was stirred with ice-cooling for 20 minutes and a solution of 2-methoxybenzyl chloride (0.92 g, 5.87 mmoles) in dimethylformamide (1 ml) was added dropwise thereinto. The mixture was stirred at room temperature for 12 hours, then at 60°C for 24 hours. After cooling, the reaction mixture was concentrated and the resulting residue was partitioned between ethyl acetate and an aqueous solution of ammonium chloride. The aqueous layer was extracted with ethyl acetate. The combined extracts were washed with an aqueous solution of sodium chloride, dried over $MgSO_4$ and evaporated in vacuo. The resulting residue was chromatographed on silica gel to give a pale yellow solid (0.70 g; 56%), which was recrystallized from ethyl acetate-hexane to give colorless powders.

m.p. 175-177°C .

| Elemental analysis (%) for $C_{19}H_{19}N_3O_7S$: | | | |
|---|---|---|---|
| Calcd. | C 52.65 | H 4.42, | N 9.69; |
| Found | C 52.87, | H 4.33, | N 9.50. |

$^1$H-NMR (200MHz, $CDCl_3$) $\delta$ : 1.31 (3H, t, J = 7.2Hz), 2.97 (3H, s), 3.90 (3H, s), 4.26 (2H, q, J = 7.1Hz), 4.79 (2H, s), 5.24 (2H, s), 6.90-6.97 (2H, m), 7.17-7.37 (2H, m).

IR (KBr): 2970, 1740, 1721, 1678, 1547, 1489 $cm^{-1}$.

Reference Example 9.

Production of ethyl 2,4(1H,3H)-dioxo-1-(2-methylthiobenzyl)-5-methyl-6-nitrothieno[2,3-d]pyrimidine-3-acetate.

The same operations as in Reference Example 8 were carried out by using 2-methylthiobenzyl chloride instead of 2-methoxybenzyl chloride to give pale yellow powders (1.33 g; 62%).

$^1$H-NMR (200MHz, $CDCl_3$) $\delta$ : 1.31 (3H, t, J = 7.1Hz), 2.57 (3H, s), 2.98 (3H, s), 4.27 (2H, q, J = 7.1Hz), 4.82 (2H, s), 5.36 (2H, s), 6.99-7.36 (4H, m).

Reference Example 10.

Production of ethyl 2,4(1H,3H)-dioxo-1-(2-methylthiobenzyl)-5-methyl-6-acetamidothieno[2,3-d]pyrimidine-3-acetate.

The compound produced in Reference Example 9 (1.10 g; 2.45 mmoles) was dissolved in acetic acid (50 ml). To this solution was added iron powder (0.72 g, 12.3 mmoles) and the resulting mixture was stirred at 80°C for four hours. The reaction mixture was concentrated and the resulting residue was partitioned between ethyl acetate and a saturated aqueous solution of sodium bicarbonate. The aqueous layer was extracted with ethyl acetate. The combined extracts were washed with an aqueous solution of sodium chloride, dried over $MgSO_4$ and evaporated in vacuo. The resulting residue was chromatographed on silica gel to give a pale yellow solid (1.2 g), which was recrystallized from ethyl acetate-hexane to give yellow plates (0.79 g; 70%).

$^1$H-NMR (200MHz, $CDCl_3$) $\delta$ : 1.31 (3H, t, J = 7.1Hz), 2.16 (3H, s), 2.37 (3H, s), 4.26 (2H, s), 4.82 (2H, q, J = 7.1Hz), 5.30 (2H, s), 6.96-7.36 (4H, m), 7.87 (1H, s).

Reference Example 11.

Production of ethyl 2,4(1H,3H)-dioxo-1-(2-methoxybenzyl)-5-methyl-6-acetamidothieno[2,3-d]pyrimidine-3-acetate.

The compound prepared in Reference Example 8 was treated by the same manner as in Reference Example 10 to give colorless powders (0.09 g; 29%).

m.p. 233-234°C .

| Elemental analysis (%) for $C_{21}H_{23}N_3O_6S \cdot 0.1H_2O$: | | | |
|---|---|---|---|
| Calcd. | C 56.39, | H 5.23, | N 9.39; |
| Found | C 56.30, | H 5.18, | N 9.34. |

$^1$H-NMR (200MHz, $CDCl_3$) δ : 1.32 (3H, t, J = 7.1Hz), 2.16 (3H, s), 2.34 (3H, s), 3.91 (3H, s), 4.27 (2H, q, J = 7.1Hz), 4.80 (2H, s), 5.18 (2H, s), 6.81-6.90 (2H, m), 7.04-7.08 (1H, m), 7.20-7.28 (1H, m), 8.11 (1H, s).

IR (KBr): 3296, 1760, 1700, 1644, 1586, 1553, 1493 $cm^{-1}$.

Reference Example 12.

Production of ethyl 2,4(1H,3H)-dioxo--6-bromo-5-methylthieno[2,3-d]pyrimidine-3-acetate.

A mixture of the compound produced in Reference Example 6 (3.50 g, 13.05 mmoles), N-bromosuccinimide (2.55 g, 14.35 mmoles) and chloroform (360 ml) was refluxed for 3 hours. After cooling, the reaction mixture was partitioned between chlorform and an aqueous solution of sodium chloride. The aqueous layer was extracted with chloroform. The combined extracts were dried over $MgSO_4$ and evaporated in vacuo. The resulting residue was recrystallized from ethyl acetate-hexane to give colorless crystals (3.15 g, 67%).

m.p. 189-190°C .

$^1$H-NMR (200MHz, $CDCl_3$) δ : 1.34 (3H, t, J = 7.2Hz), 2.40 (3H, s), 4.28 (2H, q, J=7.2Hz), 4.76 (2H, s), 10.20 (1H, s).

IR (KBr): 1748, 1717, 1657, 1572, 1437 $cm^{-1}$.

Reference Example 13.

Production of ethyl 2,4(1H,3H)-dioxo-6-bromo-5-methyl-1-(2-methylthiobenzyl)thieno[2,3-d]pyrimidine-3-acetate.

To a solution of the compound produced in Refernce Exapmple 12 (2.00 g, 5.76 mmoles) in dimethylformamide (30 ml) was added potassium carbonate (1.19 g, 8.64 mmoles), potassium iodide (0.19 g, 1.15mmoles) and 2-methylthiobenzyl chloride (1.99 g, 11.52 mmoles) and the mixture was stirred at room temperature for 16 hours. The reaction mixture was concentrated and the resulting residue was partitioned between ethyl acetate and an aqueous solution of sodium chloride. The aqueous layer was extracted with ethyl acetate. The combined extracts were dried over $MgSO_4$ and evaporated in vacuo. The resulting residue was chromatographed on silica gel to give white powders (2.12 g, 76%).

m.p. 150-151°C

$^1$H-NMR (200MHz, $CDCl_3$) δ : 1.30 (3H, t, J = 7.1Hz), 2.44 (3H, s), 2.55 (3H, s), 4.24 (2H, q, J = 7.1Hz), 4.82 (2H, s), 5.29 (2H, s), 6.97-7.34 (4H, m).

IR (KBr): 1746, 1707, 1669, 1475 $cm^{-1}$.

Example 1.

Production of ethyl 2,4(1H,3H)-dioxo-6-phenylthieno[2,3-d]pyrimidine-3-acetate (Compound 1).

Ethyl isocyanoacetate (1.4 ml; 12.5 mmoles) was added dropwise into a solution of the compound produced in Reference Example 1 (2.0 g, 8.09 mmoles) in pyridine (8 ml) and the mixture was stirred at 45°C for 2 hours. The reaction mixture was concentrated in vacuo, the resulting residue was suspended in a mixture of ethanol (20 ml) and sodium ethoxide [prepared from metal sodium (0.38 g, 16.5 mmoles) and ethanol (15 ml)]. The mixture was stirred at room temperature for 3 hours. To the reaction mixture was added 2N hydrochloric acid (10 ml) with ice-cooling and ethanol was evaporated therefrom in vacuo. The resulting solids were washed with water-ethanol and dried over phosphorus pentaoxide to give colorless powders (2.57 g; 96%), which was recrystallized from ethanol to afford colorless crystals.

m.p. 279-280.5°C.

| Elemental analysis (%) for $C_{16}H_{14}N_2O_4S \cdot 0.2H_2O$: | | | |
|---|---|---|---|
| Calcd. | C 57.54, | H 4.35, | N 8.39; |
| Found | C 57.56, | H 4.32, | N 8.36. |

$^1H$-NMR (200MHz $CDCl_3$-DMSO-$d_6$) δ : 1.29 (3H, t, J = 7.2 Hz), 4.23 (2H, q, J = 7.2Hz), 4.75 (2H, s), 7.26-7.55 (6H, m), 12.08 (1H, s).
IR (KBr): 3140, 2986, 1745, 1725, 1659, 1566, 1547, 1483 $cm^{-1}$.

Example 2.

The compounds listed in Table 1 were produced starting from the compounds obtained in Reference Examples 1, 2 and 3 by the same method as mentioned in Example 1.

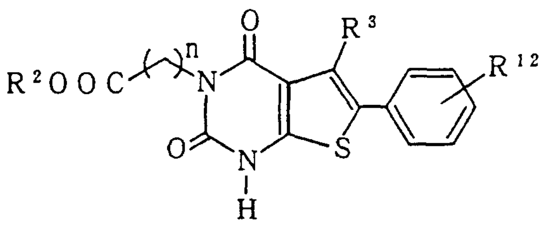

Table 1

| Cpd. No. | $R^3$ | $R^{12}$ | $R^2$ | n | Yield (%) | M.p. (°C) |
|---|---|---|---|---|---|---|
| 2 | Hydrogen | Hydrogen | Ethyl | 2 | 65 | 231-233 |
| 3 | Hydrogen | Hydrogen | Methyl | 3 | 43 | 214-215 |
| 4 | Methyl | Hydrogen | Ethyl | 1 | 41 | 119-120 |
| 5 | Methyl | 4-Methoxy | Ethyl | 1 | 96 | 164-165 |
| 6 | Methyl | 4-Methoxy | Ethyl | 2 | 84 | 185-186 |
| 7 | Methyl | 4-Methoxy | Methyl | 3 | 81 | 179-180 |
| 8 | Methyl | 3,4-Methylenedioxy | Ethyl | 1 | 88 | 204-205 |
| 9 | Methyl | 3,4-Dimethoxy | Ethyl | 1 | 90 | 220-221 |

Compound 5 in Table 1 was also produced by the following method.
A solution of a compound prepared in Reference Example 2 (0.58g, 2.00 mmoles) and triphosgene (0.42g, 1.40 mmoles) in dioxan (10 ml) was stirred at 100°C for 3 hours. The reaction mixture was concentrated to dryness. To the residue were added ethylglycine (0.51g, 5.00 mmoles) and pyridine (10 ml) and the mixture was stirred at room temperature for 30 minutes. The reaction mixture was concentrated and the resulting residue was partitioned between dichloromethane and an diluted hydrochloric acid. The aqueous layer was extracted with dichloromethane. The combined extracts were dried over $MgSO_4$ and evaporated in vacuo. The resulting residue was suspended in a mixture of ethanol (5 ml) and sodium ethoxide [prepared from metal sodium (0.07 g, 3.00 mmoles) and ethanol (3 ml)]. The mixture was stirred at room temperature for 3 hours. To the reaction mixture was added 2N hydrochloric acid (4 ml) with ice-cooling and ethanol was evaporated therefrom in vacuo. The resulting crystals were washed with aqueous ethanol and dried over phosphorus pentaoxide to give colorless powders (0.53 g; 71%).

Example 3.

Production of ethyl 2,4(1H,3H)-dioxo-5-methyl-6-(4-nitrophenyl)thieno[2,3-d]pyrimidine-3-acetate (Compound 10).

A solution of sodium nitrate (0.125 g, 1.45 mmoles) in concentrated sulfuric acid (3.5 ml) was added dropwise into a solution of the compound 4 produced in Example 2 (0.50 g, 1.45 mmloes) in concentrated sulfuric acid (3 ml) with ice-cooling. The mixture was stirred with ice-cooling for one hour. The reaction mixture was poured into ice-water and the mixture was extracted with ethyl acetate. The extract was washed with an aqueous solution of sodium chloride, dried over $MgSO_4$ and evaporated in vacuo. The resulting residue was recrystallized from ethyl acetate to give yellow crystals (0.285 g; 50%).
m.p. 276-278°C.

| Elemental analysis (%) for $C_{17}H_{15}N_3O_6S$: | | | |
|---|---|---|---|
| Calcd. | C 52.44, | H 3.88, | N 10.79; |
| Found | C 52.63, | H 3.76, | N 10.61. |

$^1$H-NMR (200 MHz, DMSO-$d_6$) δ : 1.22 (3H, t, J = 7.1Hz), 2.52 (3H, s), 4.16 (2H, q, J = 7.1Hz), 4.61 (2H, s), 7.77 (2H, d, J = 8.9Hz), 8.31 (2H, d, J = 8.9Hz), 12.61 (1H, brs).

IR (KBr): 2928, 1748, 1721, 1659, 1597, 1568, 1520, 1460, 1348 $cm^{-1}$.

Example 4.

Production of ethyl 2,4(1H,3H)-dioxo-6-(4-hydroxyphenyl)-5-methylthieno[2,3-d]pyrimidine-3-acetate (Compound 11).

A solution of the compound produced in Example 2 (2.0 g, 5.34 mmoles) in dichloromethane (40 ml) was added dropwise into a mixture of anhydrous aluminum chloride (2.90 g, 21.7 mmoles), methyl sulfide (2.45 ml, 27.2 mmoles) and dichloromethane (60 ml) with ice-cooling. The mixture was stirred at room temperature for 20 hours. The reaction mixture was poured into ice-water and the mixture was evaporated in vacuo. The resulting suspension was extracted with ethyl acetate. The extract was washed with an aqueous solution of sodium chloride, dried over $MgSO_4$ and evaporated in vacuo. The resulting residue was chromatographed on silica gel to give colorless powders (1.64 g; 85%). The resulting powders were recrystallized from ethyl acetate to give colorless crystals.

m.p. 240-242°C .

| Elemental analysis (%) for $C_{17}H_{16}N_2O_5S \cdot 0.1H_2O$: | | | |
|---|---|---|---|
| Calcd. | C 56.38, | H 4.51, | N 7.73; |
| Found | C 56.28, | H 4.48, | N 7.64. |

$^1$H-NMR (200MHz, DMSO-$d_6$) δ : 1.22 (3H, t, J = 7.1Hz), 2.37 (3H, s), 4.15 (2H, q, J = 7.1Hz), 4.59 (2H, s), 6.85 (2H, d, J = 8.6Hz), 7.26 (2H, d, J = 8.6Hz), 9.73 (1H, s), 12.39 (1H, s).

IR (KBr): 3356, 2992, 1720, 1690, 1667, 1611, 1593, 1568, 1537, 1502 $cm^{-1}$.

Example 5.

Production of ethyl 2,4(1H,3H)-dioxo-1-(2-methoxybenzyl)-6-phenylthieno[2,3-d]pyrimidine-3-acetate (Compound 12).

A solution of the compound 1 produced in Example 1 (0.50 g, 1.51 mmoles) in dimethylformamide (6 ml) was added dropwise into a suspension of sodium hydride (61 mg, 1.53 mmoles) in dimethylformamide (3 ml) with ice-cooling under nitrogen atmosphere. The mixture was stirred with ice-cooling for 20 minutes and a solution of 2-methoxybenzyl chloride (0.72 g, 4.60 mmoles) in dimethylformamide (3 ml) was added dropwise to the mixture. The mixture was stirred at room temperature for 22 hours and then concentrated. The resulting residue was partitioned between ethyl acetate-tetrahydrofuran and an aqueous solution of ammonium chloride. The aqueous layer was extracted with ethyl acetate. The combined extracts were washed with an aqueous solution of sodium chloride, dried over $MgSO_4$ and evaporated in vacuo. The resulting residue was chromatographed on silica gel to give pale yellow powders (0.463 g; 68%). The resulting powders were recrystallized from ethyl acetate to give colorless crystals.

m.p 182-183.5°C .

| Elemental analysis (%) for $C_{24}H_{22}N_2O_5S$: | | | |
|---|---|---|---|
| Calcd. | C 63.99, | H 4.92, | N 6.22; |
| Found | C 63.82, | H 4.96, | N 6.25. |

$^1$H-NMR (200MHz, $CDCl_3$) δ : 1.30 (3H, t, J = 7.1Hz), 3.90 (3H, s), 4.25 (2H, q, J = 7.2Hz), 4.83 (2H, s), 5.27 (2H, s), 6.88-6.95 (2H, m), 7.14-7.18 (1H, m), 7.25-7.51 (6H, m), 7.53 (1H, s).

IR (KBr): 2996, 1750, 1709, 1667, 1557, 1526, 1499, 1473 $cm^{-1}$.

Example 6.

Production of ethyl 2,4(1H,3H)-dioxo-1-(2,4-dimethoxybenzyl)-6-(4-methoxyphenyl)-5-methylthieno[2,3-d]pyrimidine-3-acetate (Compound 13).

To a solution of the compound 5 produced in Example 2 (0.73 g, 1.95 mmoles) in anhydrous tetrahydrofuran (THF, 25 ml) were added dropwise 2,4-dimethoxybenzyl alcohol (0.49 g, 2.91 mmoles) and tri-n-butylphosphine (0.91 ml, 3.65 mmoles) and the mixture was stirred. To this solution was added azidocarbonyl dipiperidine (0.92 g, 3.55 mmoles) under nitrogen stream. The mixture was stirred under a nitrogen stream at room temperature for six hours and then at 60°C for 18 hours. After cooling, the reaction mixture was partitioned between ethyl acetate and an aqueous solution of sodium chloride. The aqueous layer was extracted with ethyl acetate. The combined extracts were washed with an aqueous solution of sodium chloride again, dried over $MgSO_4$ and evaporated in vacuo. The resulting residue was chromatographed on silica gel to give colorless powders (0.37 g; 27%). The resulting powders were recrystallized from ethyl ether-n-hexane to give colorless crystals.

m.p. 110-111°C.

| Elemental analysis (%) for $C_{27}H_{28}N_2O_7S$: | | | |
|---|---|---|---|
| Calcd. | C 61.82, | H 5.38, | N 5.34; |
| Found | C 61.80, | H 5.42, | N 5.25. |

$^1$H-NMR (200MHz, $CDCl_3$) $\delta$ : 1.30 (3H, t, J = 7.2 Hz), 2.48 (3H, s), 3.77 (3H, s), 3.83 (6H s), 4.25 (2H, q, J = 7.2Hz), 4.82 (2H, s), 5.16 (2H, s), 6.40-6.45 (2H, m), 6.93 (2H, d, J = 8.9H), 7.07-7.11 (1H, m), 7.28 (2H, d, J = 8.9HZ).

IR (KBr): 2974, 1754, 1711, 1663, 1613, 1589, 1528, 1510, 1477 $cm^{-1}$.

Example 7.

The compounds listed in Tables 2, 3, 4 and 5 were produced by the same method as mentioned in Example 5 starting from the compounds produced in Examples 1, 2, 3, 17, 18, 19, 27 and 29.

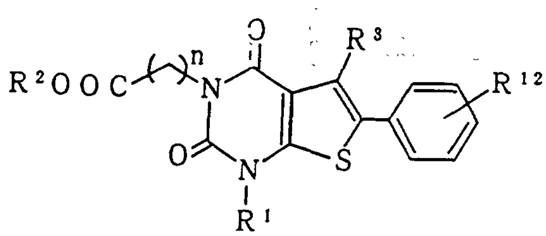

Table 2.

| Cpd. No. | $R^3$ | $R^{12}$ | $R^1$ | $R^2$ | n | Yield (%) | M.p. (°C) |
|---|---|---|---|---|---|---|---|
| 14 | H | H | 2-(1-Methylindol-3-yl)ethyl | Et | 1 | 15 | 226-227.5 |
| 15 | H | H | 2-Quinolylmethyl | Et | 1 | 56 | 199.5-201.5 |
| 16 | H | H | 3,4-Methylenedioxybenzyl | Et | 1 | 84 | 182-184 |
| 17 | H | H | 2-Methoxybenzyl | Et | 2 | 78 | 142-143 |
| 18 | H | H | 2-Methoxybenzyl | Me | 3 | 69 | 126-127 |
| 19 | H | H | Cyclohexylmethyl | Et | 1 | 75 | 169-170 |
| 20 | H | H | 3-Methoxybenzyl | Et | 1 | 64 | 164-165 |
| 21 | H | H | 4-Methoxybenzyl | Et | 1 | 91 | 143-145 |
| 22 | H | H | 2-Methoxyphenethyl | Et | 1 | 63 | 143-144 |
| 23 | Me | H | 2-Methoxybenzyl | Et | 1 | 89 | 151-152 |
| 24 | Me | H | 2-(1-Methylindol-3-yl)ethyl | Et | 1 | 91 | 179-181 |
| 25 | Me | 4-MeO | 2-Methoxybenzyl | Et | 1 | 78 | 141-142 |

(Me: methyl; Et: ethyl; MeO: methoxy)

Table 3

| Cpd. No. | $R^3$ | $R^{12}$ | $R^1$ | $R^2$ | n | Yield (%) | M.p. (°C ) |
|---|---|---|---|---|---|---|---|
| 26 | Me | 4-MeO | 2(1-Methylindol-3-yl)ethyl | Et | 1 | 85 | 163-164 |
| 27 | Me | 4-$NO_2$ | 2-Methoxybenzyl | Et | 1 | 87 | 178-179 |
| 28 | Me | 4-MeO | Butyl | Et | 1 | 81 | 134.5-136 |
| 29 | Me | 4-MeO | Benzyl | Et | 1 | 81 | 126-128 |
| 30 | Me | 4-MeO | 2-Methylbenzyl | Et | 1 | 78 | 127-128.5 |
| 31 | Me | 4-MeO | 2-Bromobenzyl | Et | 1 | 94 | 144-144.5 |
| 32 | Me | 4-MeO | 2-Nitrobenzyl | Et | 1 | 62 | 154-156 |
| 33 | Me | 4-MeO | 2-Cyanobenzyl | Et | 1 | 80 | 176-177 |
| 34 | Me | 4-MeO | 2-Methylthiobenzyl | Et | 1 | 88 | 144-145 |
| 35 | Me | 4-MeO | 2-Methoxyphenethyl | Et | 1 | 81 | 113-115 |
| 36 | Me | 4-Nitro | 2-Methylthiobenzyl | Et | 1 | 79 | 152-153 |

(Me: methyl; Et: ethyl; MeO: methoxy)

Table 4.

| Cpd. No. | $R^3$ | $R^{12}$ | $R^1$ | $R^2$ | n | Yield (%) | M.p. (°C) |
|---|---|---|---|---|---|---|---|
| 37 | Me | 4-MeO | 1-Naphthylmethyl | Et | 1 | 76 | 167-170 |
| 38 | Me | 4-MeO | 2-Naphthylmethyl | Et | 1 | 95 | 130.5-132 |
| 39 | Me | 2-MeO | 2-Methoxybenzyl | Et | 1 | 90 | 147-148 |
| 40 | Me | 4-MeO | 2-Methoxybenzyl | Et | 2 | 83 | 156-157 |
| 41 | Me | 4-MeO | 2-Methoxybenzyl | Me | 3 | 93 | 139-140 |
| 42 | Me | 4-MeO | 3-Methoxybenzyl | Et | 1 | 91 | 144-145 |
| 43 | Me | 4-MeO | 4-Methoxybenzyl | Et | 1 | 87 | 167-168 |
| 44 | Me | 4-MeO | 2,3-Dimethoxybenzyl | Et | 1 | 86 | 135-137 |
| 45 | Me | 4-MeO | 2,5-Dimethoxybenzyl | Et | 1 | 76 | 180-181 |
| 46 | Me | 3,4-Methylenedioxy | 2-Methylthiobenzyl | Et | 1 | 87 | 145-147 |
| 47 | Me | 3,4-Dimethoxy | 2-Methylthiobenzyl | Et | 1 | 77 | 144-145 |
| 48 | Me | 4-MeO | 2-Hydroxybenzyl | Et | 1 | 84 | 183-184 |
| 49 | Me | 4-MeO | 2-Ethoxybenzyl | Et | 1 | 71 | 138-139 |
| 50 | Me | 4-MeO | 2-Benzyloxybenzyl | Et | 1 | 76 | 120-127 |
| 51 | Me | 4-MeO | 2'-Cyanobiphenylmethyl | Et | 1 | 93 | 187-188 |
| 52 | Me | 4-MeO | 2-Methoxymethoxybenzyl | Et | 1 | 63 | 108-109 |
| 53 | Me | 4-MeO | 2-Benzimidazolmethyl | Et | 1 | 82 | amorphous |
| 54 | Me | 4-MeO | 2-Picolyl | Et | 1 | 80 | 132-133 |
| 55 | Me | 4-MeO | 2-Fluorobenzyl | Et | 1 | 77 | 125-127 |
| 56 | Me | 4-Butyryl | 2-Methylthiobenzyl | Et | 1 | 91 | 124-125 |
| 57 | Me | 4-Ethythiomethyl | 2-Methylthiobenzyl | Et | 1 | 76 | amorphous |

(Me: methyl; Et: ethyl; MeO: methoxy)

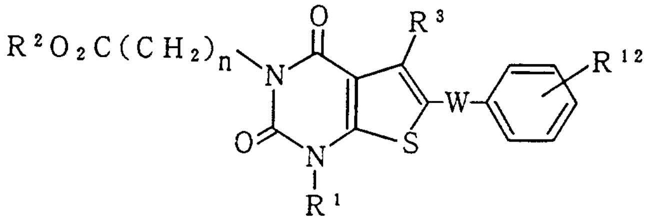

Table 5.

| Cpd. No. | $R^3$ | W | $R^{12}$ | $R^1$ | $R^2$ | n | Yield (%) | M.p. (°C ) |
|---|---|---|---|---|---|---|---|---|
| 58 | Me | Sulfonylpiperazinyl | H | 2-Methoxybenzyl | Et | 1 | 93 | amorphous |
| 59 | Me | Carbonyl | H | 2-Methylthiobenzyl | Et | 1 | 88 | 127-128 |
| 60 | Me | Carbonyl | 4-MeO | 2-Methylthiobenzyl | Et | 1 | 94 | 128-129 |
| (Me: methyl; Et: ethyl; Meo: methoxy) | | | | | | | | |

Example 8.

Production of 2,4(1H,3H)-dioxo-1-(2-methylsulfinylbenzyl)-6-(4-methoxyphenyl)-5-methylthieno[2,3-d]pyrimidine-3-acetate (Compound 61).

A solution of m-chloroperbenzoic acid (0.26 g, 0.753 mmole) in dichloromethane (10 ml) was added dropwise into a solution of the compound produced in Example 7 (0.35 g, 0.685 mmole) in dichloromethane (10 ml) with ice-cooling. After the addition was complete, the mixture was further stirred at 0°C for 30 minutes. The reaction mixture was poured into a 5% aqueous solution of sodium bicarbonate, the aqueous layer was extracted with ethyl acetate. The combined organic layers were washed with an aqueous solution of sodium chloride, dried over $MgSO_4$ and evaporated in vacuo. The resulting residue was chromatographed on silica gel to give colorless amorphous crystals (0.26 g; 72%).

m.p. 90-95°C .

| Elemental analysis (%) for $C_{26}H_{26}N_2O_6S_2 \cdot 0.2H_2O$: | | | |
|---|---|---|---|
| Calcd. | C 58.89, | H 5.01, | N 5.28; |
| Found | C 58.89, | H 5.04, | N 5.22. |

$^1$H-NMR (200MHz, DMSO-$d_6$) δ : 1.31 (3H, t, J = 7.1Hz), 2.50 (3H, s), 2.83 (3H, s), 3.83 (3H, s), 4.25 (2H, q, J = 7.1Hz), 4.82 (2H, s), 5.19 (1H, d, J = 16.2Hz), 5.49 (1H, d, J = 16.2Hz), 6.93 (2H, d, J = 8.8Hz), 7.23-7.57 (5H, m), 8.05 (1H, d, J = 7.8Hz).

IR (KBr): 1748, 1709, 1665, 1609, 1564, 1535 $cm^{-1}$.

Example 9.

Production of ethyl 2,4(1H,3H)-dioxo-1-(2-methylsulfonylbenzyl)-6-(4-methoxyphenyl)-5-methylthieno[2,3-d]pyrimidine-3-acetate (Compound 62).

A solution of m-chloroperbenzoic acid (0.52 g, 1.51 mmoles) in dichloromethane (10 ml) was added dropwise into a solution of the compound 34 produced in Example 6 (0.35 g, 0.685 mmole) in dichloromethane (10 ml) with ice-cooling. After the addition was complete, the mixture was stirred at 0°C for one hour and then at room temperature for 4 hours. The reaction mixture was poured into a 5% aqueous solution of sodium bicarbonate, the aqueous layer was extracted with ethyl acetate. The combined organic layers were washed with an aqueous solution of sodium chloride, dried over $MgSO_4$ and evaporated in vacuo. The resulting residue was chromatographed on silica gel to give a pale yellow solid (0.37 g; 99%), which was recrystallized from ethyl acetate-hexane to give 0.25 g (67%) of colorless crystals.

m.p. 141-144°C .

| Elemental analysis (%) for $C_{26}H_{26}N_2O_7S_2 \cdot H_2O$: | | | |
|---|---|---|---|
| Calcd. | C 57.55, | H 4.83, | N 5.16: |
| Found | C 57.64, | H 4.69, | N 5.16. |

$^1$H-NMR (200 MHz, DMSO-$d_6$) δ : 1.28 (3H, t, J = 7.1Hz), 2.53 (3H, s), 3.26 (3H, s), 3.84 (3H, s), 4.23 (2H, q, J = 7.1Hz), 4.79 (2H, s), 5.64 (2H, s), 6.94 (2H d, J = 8.9Hz), 7.22-7.32 (3H, m), 7.48-7.64 (2H, m), 8.11 (1H, d, J = 7.6Hz).

IR (KBr): 1742, 1705, 1661, 1533, 1477, 1377 $cm^{-1}$.

Example 10.

Production of ethyl 5-bromomethyl-2,4(1H,3H)-dioxo-1-(2-methoxybenzyl)-6-phenylthieno[2,3-d]pyrimidine-3-acetate (Compound 63).

A mixture of the compound 23 produced in Example 7 (0.20 g, 0.431 mmole), N-bromosuccinimide (80 mg, 0.45 mmole), α,α'-azobisisobutyronitrile (7 mg, 0.043 mmole) and carbon tetrachloride (5 ml) was refluxed for two hours. After cooling, the insoluble matter was filtered off and the filtrate was diluted with chloroform. The organic layer was washed with an aqueous solution of sodium chloride, dried over $MgSO_4$ and evaporated in vacuo. The resulting residue was chromatographed on silica gel to give a solid, which was crystallized from ether-hexane to give colorless crystals (0.204 g; 87%).

m.p. 123-124°C .

| Elemental analysis (%) for $C_{25}H_{23}N_2O_5SBr$: | | | |
|---|---|---|---|
| Calcd. | C 55.25, | H 4.27, | N 5.15; |
| Found | C 55.04, | H 4.21, | N 5.01. |

$^1$H-NMR (200MHz, $CDCl_3$) δ : 1.30 (3H, t, J = 7.1HZ), 3.87 (3H, s), 4.25 (2H, q, J = 7.1Hz), 4.80 (2H, s), 4.86 (2H, s), 5.26 (2H, s), 6.88-6.96 (2H, m), 7.15-7.19 (1H, m), 7.25-7.58 (6, m).

IR (KBr): 2974, 1754, 1713, 1663, 1555, 1531, 1493, 1479 $cm^{-1}$.

Example 11.

Production of ethyl 2,4(1H,3H)-dioxo-1-(2-methoxybenzyl)-5-{(N-methyl-N-phenyl)aminomethyl}-6-phenylthieno [2,3-d]pyrimidine-3-acetate (Compound 64).

To a solution of the compound 60 produced in Example 10 (0.72 g, 1.32 mmoles) in dimethylformamide (6 ml) was added triethylamine (0.28 ml, 2.0 mmoles) followed by adding N-methylaniline (0.22 ml, 2.03 mmoles) thereto. The mixture was stirred at room temperature for 4 hours. The reaction mixture was concentrated and the resulting residue was partitioned between ethyl acetate and an aqueous solution of sodium chloride. The aqueous layer was extracted with ethyl acetate. The combined organic layers were washed with an aqueous solution of sodium chloride, dried over $MgSO_4$ and evaporated in vacuo. The resulting residue was chromatographed on silica gel to give white amorphous powders (0.45 g; 60%).

$^1$H-NMR (200MHz, $CDCl_3$) δ : 1.30 (3H t, J = 7.2Hz), 2.60 (3H, s), 3.86 (3H, s), 4.25 (2H, q, J = 7.2Hz), 4.72 (2H, s), 4.84 (2H, s), 5.26 (2H, s), 6.65-6.72 (3H, m), 6.88-6.96 (2H, m), 7.10-7.37 (9H, m).

IR (KBr): 2966, 1752, 1711, 1667, 1601, 1560, 1531, 1493, 1473 $cm^{-1}$.

Example 12.

Production of ethyl 2,4(1H,3H)-dioxo-6-(4-hydroxyphenyl)-1-(2-methoxybenzyl)-5-methylthieno[2,3-d]pyrimidine-3-acetate (Compound 65).

Acetic anhydride (3 ml; 31.8 mmoles) was added to a solution of the compound 11 produced in Example 4 (0.60 g, 1.66 mmoles) in pyridine (8 ml) and the mixture was stirred at room temperature for three hours. The reaction mixture was concentrated and the residue was partitioned between ethyl acetate and diluted hydrochloric acid. The aqueous layer was extracted with ethyl acetate. The combined organic layers were washed with an aqueous solution of sodium chloride, dried over $MgSO_4$ and evaporated in vacuo. The residue was chromatographed on silica gel to give white amorphous powders (0.57 g). To a solution of the resulting amorphous powders in dimethylformamide (5 ml) were added potassium carbonate (0.38 g, 2.75 mmoles) and 2-methoxybenzyl chloride (0.65 g, 4.15 mmoles). The mixture was stirred at room temperature for 22 hours. The reaction mixture was concentrated and the residue was partitioned between ethyl acetate and an aqueous solution of sodium chloride. The aqueous layer was extracted with ethyl acetate. The combined organic layers were washed with an aqueous solution of sodium chloride, dried over $MgSO_4$ and was evaporated in vacuo. The residue was chromatographed on silica gel to give amorphous powders (0.60 g). The amorphous powders were dissolved in a mixture of methanol (18 ml) and tetrahydrofuran (12 ml). To this solution was added dropwise a solution of potassium carbonate (0.313 g, 2.26 mmoles) in water (8 ml). The mixture was stirred at room temperature for 30 minutes, and then 1N hydrochloric acid (5 ml) was added thereto with ice-cooling. The mixture was extracted with ethyl acetate. The extract was washed with an aqueous solution of sodium

chloride, dried over $MgSO_4$ and evaporated in vacuo. The residue was crystallized from ether to give colorless crystals (0.496 g; 62%).

m.p. 207-208°C.

| Elemental analysis (%) for $C_{25}H_{24}N_2O_6S$: | | | |
|---|---|---|---|
| Calcd. | C 62.49, | H 5.03, | N 5.83; |
| Found | C 62.50, | H 5.21, | N 5.85. |

$^1$H-NMR (200MHz, $CDCl_3$) δ : 1.32 (3H, t, J = 7.1Hz), 2.43 (3H, s), 3.85 (3H, s), 4.27 (2H, q, J = 7.1Hz), 4.85 (2H, s), 5.19 (2H, s), 5.78 (1H, s), 6.74-6.91 (4H, m), 7.05-7.11 (3H, m), 7.21-7.30 (1H, m).

IR (KBr): 3350, 2976, 1756, 1698, 1649, 1613, 1566, 1537, 1483 $cm^{-1}$.

Example 13.

Production of ethyl 2,4(1H,3H)-dioxo-6-(4-hydroxyphenyl)-1-(2-methylthiobenzyl)-5-methylthieno[2,3-d]pyrimidine-3-acetate (Compound 66).

Acetic anhydride (3 ml; 31.8 mmoles) was added to a solution of the compound 11 produced in Example 4 (0.60 g, 1.66 mmoles) in pyridine (8 ml) and the mixture was stirred at room temperature for three hours. The reaction mixture was concentrated and the residue was partitioned between ethyl acetate and diluted hydrochloric acid. The aqueous layer was extracted with ethyl acetate. The combined organic extracts were washed with an aqueous solution of sodium chloride, dried over $MgSO_4$ and evaporated in vacuo. The residue was chromatographed on silica gel to give a white amorphous (0.57 g). To a solution of the resulting amorphous in dimethylformamide (5 ml) were added potassium carbonate (0.38 g, 2.75 mmoles) and 2-methylthiobenzyl chloride (0.65 g, 4.15 mmoles). The mixture was stirred at room temperature for 22 hours. The reaction mixture was concentrated and the residue was partitioned between ethyl acetate and an aqueous solution of sodium chloride. The aqueous layer was extracted with ethyl acetate. The combined organic extracts were dried over $MgSO_4$ and evaporated in vacuo. The residue was chromatographed on silica gel to give a white amorphous (0.60g). The amorphous was dissolved in a mixture of methanol (18 ml) and tetrahydrofuran (12 ml) and a solution of potassium carbonate (0.313 g, 2.26 mmoles) in water (8 ml) was added dropwise thereinto. The mixture was stirred at room temperature for 30 minutes. To the reaction mixture was added dropwise 1N hydrochloric acid (5 ml) with ice-cooling and the mixture was extracted with ethyl acetate. The extract was washed with an aqueous solution of sodium chloride, dried over $MgSO_4$ and evaporated in vacuo. The residue was crystallized from ether to give colorless crystals (4.33 g; 78%).

m.p. 177-178°C .

| Elemental analysis (%) for $C_{25}H_{24}N_2O_5S_2 \cdot 1/10\ H_2O$: | | | |
|---|---|---|---|
| Calcd. | C 60.25, | H 4.89, | N 5.62; |
| Found | C 60.09, | H 4.66, | N 5.57. |

$^1$H-NMR (200MHz, $CDCl_3$) δ : 1.32 (3H, t, J = 7.2Hz), 2.45 (3H, s), 2.52 (3H, s), 4.28 (2H, q, J = 7.2Hz), 4.87 (2H, s), 5.28 (2H, s), 5.75 (1H, s), 6.78 (2H, d, J = 8.6Hz), 6.97-7.14 (4H, m), 7.21-7.34 (2H, m).

IR (KBr): 3346, 2978, 1752, 1700, 1651, 1613, 1591, 1564, 1535, 1481 $cm^{-1}$.

Example 14.

Production of ethyl 2,4(1H,3H)-dioxo-6-(4-ethoxyphenyl)-1-(2 methoxybenzyl)-5-methythieno[2,3-d]pyrimidine-3-acetate (Compound 67).

A solution of the compound 62 produced in Example 12 (0.15 g, 0.31 mmole) in dimethylformamide (3 ml) was added dropwise into a suspension of sodium hydride (14 mg, 0.35 mmole) in dimethylformamide (1 ml) with ice-cooling under nitrogen stream. The mixture was stirred with ice-cooling for 30 minutes. To this mixture was added dropwise iodoethane (0.13 ml, 1.63 mmoles). The mixture was stirred at room temperature for 2.5 hours. The reaction mixture was concentrated and the residue was partitioned between ethyl acetate and an aqueous solution of ammonium chloride. The aqueous layer was extracted with ethyl acetate. The combined extracts were washed with an aqueous solution of sodium chloride, dried over $MgSO_4$ and evaporated in vacuo. The resulting residue was recrystallized from ethyl acetate-hexane to give colorless crystals (0.119 g; 75%).

m.p. 133-134°C .

| Elemental analysis (%) for $C_{27}H_{28}N_2O_6S$: | | | |
|---|---|---|---|
| Calcd. | C 63.76, | H 5.55, | N 5.51; |
| Found | C 63.48, | H 5.62, | N 5.37. |

$^1H$-NMR (200MHz, $CDCl_3$) δ : 130 (3H, t, J = 7.1Hz), 1.43 (3H, t, J = 7.0Hz), 2.49 (3H, s), 3.87 (3H, s), 4.05 (2H, q, J = 7.0Hz), 4.25 (2H, q, J = 7.1H), 4.83 (2H, s), 5.24 (2H, s), 6.86-6.94 (4H, m), 7.09-7.14 (1H, m), 7.22-7.31 (3H, m).

IR (KBr): 2984, 1758, 1707, 1665, 1607, 1562, 1535, 1477 $cm^{-1}$.

Example 15.

The compounds listed in Table 6 were produced from the compound produed in Example 12 by the same method as mentioned in Example 14.

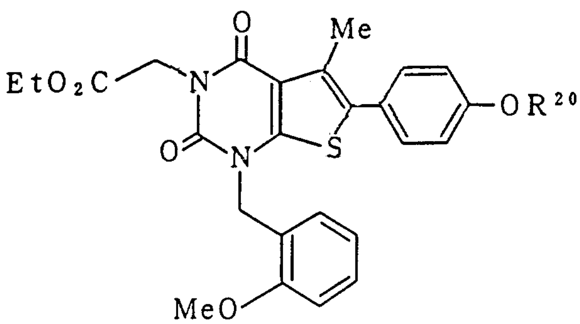

Table 6

| Cpd.No. | $R^{20}$ | Yield(%) | M.p.(°C) |
|---|---|---|---|
| 68 | 2-Methoxybenzyl | 92 | 142-143 |
| 69 | 2-Hydroxyethyl | 54 | 116-117 |
| 70 | Isopropyl | 72 | amorphous |
| 71 | Methoxymethyl | 92 | 96-97 |
| 72 | Methoxyethyl | 79 | 134-135 |
| 73 | Methylthiomethyl | 75 | 74-79 |
| 74 | Acetic Acid | 96 | 147-148 |
| 75 | Benzyl | 94 | 60-70 |
| 76 | Carbamoylmethyl | 98 | 170-171 |
| 77 | tert-Butoxycarbonyl | 100 | amorphous |
| 78 | Methylamide | 97 | 156-157 |

Example 16.

The compounds listed in Table 7 were produced from the compound produced in Example 13 by the same method as mentioned in Example 14.

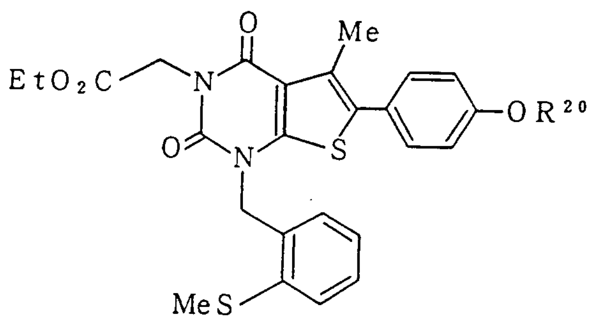

Table 7.

| Cpd.No. | $R^{20}$ | Yield (%) | M.p. (°C) |
|---|---|---|---|
| 79 | Methoxymethyl | 85 | amorphous |
| 80 | Methylthiomethyl | 77 | 110-111 |
| 81 | n-Propyl | 84 | 122-123 |
| 82 | Methoxyethoxymethyl | 89 | amorphous |
| 83 | 2-Oxopropyl | 48 | 127-128 |
| 84 | n-Butyl | 85 | 85- 88 |
| 85 | Allyl | 76 | 115-117 |
| 86 | Acetyl | 67 | amorphous |

Example 17.

Production of ethyl 2,4(1H,3H)-dioxo-6-(N'-phenylpiperazinylsulfamoyl)-5-methylthieno[2,3-d]pyrimidine-3-acetate (Compound 87).

To the compound produced in Referential Example 6 (0.54 g; 2.0 mmoles) was added chlorosulfonic acid (4 ml) and the mixture was stirred at 70°C for one hour. After cooling, the reaction mixture was poured into ice-water and extracted with ethyl acetate. The combined organic extracts were washed with an aqueous solution of sodium chloride, dried over $MgSO_4$ and evaporated in vacuo. The residue was dissolved in dimethylformamide (6 ml). To the solution were added triethylamine (0.61 ml, 4.38 mmoles) and 1-phenylpiperazine (0.34 ml, 2.23 mmoles) with ice-cooling and the mixture was stirred for one hour. The reaction mixture was concentrated and the residue was partitioned between ethyl acetate-tetrahydrofuran and diluted hydrochloric acid. The aqueous layer was extracted with ethyl acetate-tetrahydrofuran. The combined organic extracts were washed with an aqueous solution of sodium chloride, dried over $MgSO_4$ and evaporated in vacuo to give a solid (0.38 g). The resulting solid was dissolved in ethanol (30 ml). To the solution was added concentrated sulfuric acid (0.1 ml) and the mixture was refluxed for 14 hours. After cooling, the reaction mixture was partitioned between ethyl acetate and a saturated aqueous solution of sodium bicarbonate. The aqueous layer was extracted with ethyl acetate. The combined organic extracts were washed with an aqueous solution of sodium chloride, dried over $MgSO_4$ and evaporated in vacuo. The residue was chromatographed on silica gel to give a white solid (0.135 g; 14%). This was recrystallized from ethyl acetate to give colorless crystals.

m.p. 259-260°C .

| Elemental analysis (%) for $C_{21}H_{24}N_4O_6S_2$: | | | |
|---|---|---|---|
| Calcd. | C 51.21, | H 4.91, | N 11.37; |
| Found | C 51.20, | H 5.03, | N 11.26. |

$^1$H-NMR (200MHz, $CDCl_3$) δ : 1.21 (3H, t, J = 7.2Hz), 2.66 (3H, s), 3.23 (8H, s), 4.14 (2H q, J = 7.1Hz), 4.57 (2H, s), 6.77-6.94 (3H, m), 7.16-7.25 (2H, m).

IR (KBr): 3138, 1740, 1721, 1680, 1651, 1603, 1564, 1520, 1497 $cm^{-1}$.

Example 18.

Production of ethyl 2,4(1H,3H)-dioxo-6-benzoyl-5-methylthi no[2,3-d]pyrimidine-3-acetate (Compound 88).

To a suspension of the compound produced in Reference Example 6 (0.54 g, 2.0 mmoles) in nitromethane (10 ml) were added aluminum chloride (1.13 g, 8.47 mmoles) and benzoyl chloride (0.48 ml, 4.14 mmoles) under nitrogen stream and then stirred 2 hours. The mixture was stirred at 40°C for 6 hours. After cooling, the reaction mixture was poured into ice-water and extracted with ethyl acetate. The combined organic extracts were washed with an aqueous solution of sodium chloride, dried over $MgSO_4$ and evaporated in vacuo. The resulting residue was recrystallized from ether-ethyl acetate to give colorless crystals (0.56 g ; 74%).

m.p. 202-203°C.

| Elemental analysis (%) for $C_{18}H_{16}N_2O_5S$: | | | |
|---|---|---|---|
| Calcd. | C 58.06, | H 4.33, | N 7.52; |
| Found | C 57.80, | H 4.40, | N 7.37. |

$^1$H-NMR (200MHz, $CDCl_3$) δ : 1.22 (3H, t, J = 7.2Hz), 2.45 (3H, s), 4.15 (2H, q, J = 7.2Hz), 4.59 (2H, s), 7.51-7.74 (5H, m).

IR (KBr): 3190, 1734, 1709, 1678, 1609, 1557, 1553 $cm^{-1}$.

Example 19.

Production of ethyl 2,4(1H,3H)-dioxo-6-(4-methoxybenzoyl)-5-methylthieno[2,3-d]pyrimidine-3-acetate (Compound 89 ).

The same operations as in Example 18 were carried out by using 4-methoxybenzoyl chloride (0.515 g, 3.0 mmoles) instead of benzoyl chloride to give colorless plates (0.34 g; 42%).

m.p. 209-211°C.

| Elemental analysis (%) for $C_{19}H_{18}N_2O_6S \cdot 0.1\ H_2O$: | | | |
|---|---|---|---|
| Calcd. | C 56.46, | H 4.54, | N 6.93; |
| Found | C 56.36, | H 4.74, | N 6.74. |

$^1$H-NMR (200MHz, $CDCl_3$) δ : 1.31 (3H, t, J = 7.1Hz), 2.51 (3H, s), 3.90 (3H, s), 4.26 (2H, q, J = 7.1Hz), 4.76 (2H, s), 6.97 (2H, d, J = 8.9Hz), 7.77 (2H,d, J = 8.9Hz).

IR (KBr) : 3124, 2972, 1734, 1669, 1593, 1562, 1543, 1510 $cm^{-1}$.

Example 20.

Production of ethyl 2,4(1H,3H)-dioxo-1-(2-methylthiobenzyl)-6-(4-methoxybenzamido)-5-methylthieno[2,3-d] pyrimidine-3-acetate (Compound 90).

To a solution of the compound produced in Reference Example 10 (0.60 g, 1.30 mmoles) in dichloromethane (35 ml) were added 4-dimethylaminopyridine (0.027 g; 0.23 mmole) and triethylamine (0.35 ml, 2.5 mmoles). To this solution was added dropwise a solution of 4-methoxybenzoyl chloride (0.3 ml, 2.6 mmoles) in dichloromethane (5 ml). After the addition was complete, the mixture was stirred at room temperature for 1.5 hours. The reaction mixture was partitioned between dichloromethane and a saturated aqueous solution of sodium chloride. The aqueous layer was extracted with dichloromethane. The combined organic extracts were washed with an aqueous solution of sodium chloride, dried over $MgSO_4$ and evaporated in vacuo. The residue was chromatographed on silica gel to give white amorphous powders (0.46 g). The amorphous powders (0.3 g; 0.52 mmole) were dissolved in a mixture of methanol (6 ml) and tetrahydrofuran (6 ml). To the mixture was added a solution of potassium carbonate (0.15 g) in water (3 ml) and the mixture was stirred at room temperature for 10 minutes. To the reaction mixture was added 1N hydrochloric acid (1.5 ml) at 0°C and the mixture was extracted with ethyl acetate. The combined organic extracts were washed with an aqueous solution of sodium chloride, dried over $MgSO_4$ and evaporated in vacuo. The residue was chromatographed on silica gel to give white powdery crystals (0.20 g; 70%), which were recrystallized from ethyl acetate-hexane.

m.p. 199-200°C.

| Elemental analysis (%) for $C_{27}H_{27}N_3O_6S_2$: | | | |
|---|---|---|---|
| Calcd. | C 58.57, | H 4.92, | N 7.59; |
| Found | C 58.58, | H 5.06, | N 7.36. |

$^1$H-NMR (200MHz, $CDCl_3$) δ : 1.30 (3H, t, J = 7.2Hz), 2.53 (3H, s), 2.58 (3H, s), 3.88 (3H, s), 4.25 (2H, q, J = 7.2Hz), 4.84 (2H, s), 5.34 (2H, s), 6.94-7.33 (7H, m), 7.80 (1H, d).

IR (KBr): 3358, 2982, 1746, 1702, 1661, 1607, 1493 $cm^{-1}$.

Example 21.

Production of ethyl 2,4(1H,3H)-dioxo-1-(2-methoxybenzyl)-6-benzamido-5-methylthieno[2,3-d]pyrimidine-3-acetate (Compound 91).

The same operations as in Example 20 were carried out with the compound obtained in Reference Example 11 instead of 4-methoxybenzoyl chloride to give colorless powdery crystals (0.06 g; 53%).

$^1$H-NMR (200MHz, $CDCl_3$) δ : 1.29 (3H, t, J = 7.1Hz), 2.51 (3H, s), 3.91 (3H, s), 4.24 (2H, q, J = 7.1Hz), 4.81 (2H, s), 5.25 (2H, s), 6.83-6.91 (2H, m), 7.05-7.09 (1H, m) 7.21-7.30 (1H, m), 7.46-7.59 (3H, m), 7.82-7.87 (2H, m), 8.05 (1H, s).

Example 22.

Production of 2,4(1H,3H)-dioxo-1-(2-methoxybenzyl)-6-phenylthieno[2,3-d]pyrimidine-3-acetic acid (Compound 92).

The compound 12 (0.20 g; 0.44 mmole) produced in Example 5 was dissolved in a mixture of methanol (6 ml) and tetrahydrofuran (12 ml). To the solution was added dropwise 1N sodium hydroxide solution (2 ml, 2 mmoles). After stirring the mixture at room temperature for three hours, 1N hydrochloric acid (3 ml) was added to the reaction mixture with ice-cooling and the mixture was extracted with ethyl acetate. The extract was washed with an aqueous solution of sodium chloride, dried over $MgSO_4$ and evaporated in vacuo. The residue was recrystallized from ethanol to give pale yellow crystals (0.153 g; 82%).

m.p. 272-273°C.

| Elemental analysis (%) for $C_{22}H_{18}N_2O_5S$: | | | |
|---|---|---|---|
| Calcd. | C 62.55, | H 4.29, | N 6.63; |
| Found | C 62.52, | H 4.37, | N 6.89. |

$^1$H-NMR (200MHz, DMSO-$d_6$) δ : 3.88 (3H, s), 4.62 (2H, s), 5.18 (2H, s), 6.86-6.94 (1H, m), 706-7.10 (2H, m), 7.28-7.45 (4H, m),7.62-7.66 (2H, m), 7.72 (1H, s).

IR (KBr): 3128, 1730, 1710, 1659, 1560, 1526, 1497, 1475 $cm^{-1}$.

Example 23.

The compounds listed in Tables 8, 9, 10, 11 and 12 were produced by the same manner as mentioned in Example 22 from the compounds produced in Examples 2, 6, 7, 8, 9, 10, 11, 12, 13, 14, 15, 16, 19, 20, 21, 30, 31, 33, 34, 35 and 36.

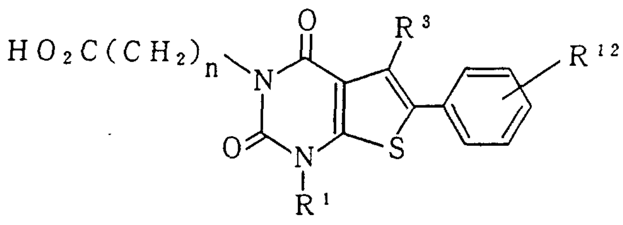

Table 8

| Cpd. No. | $R^3$ | $R^{12}$ | $R^1$ | n | Yield (%) | M.p. (°C ) |
|---|---|---|---|---|---|---|
| 93 | H | H | 2-(1-Methylindol-3-yl)ethyl | 1 | 60 | 269-271 |
| 94 | H | H | 2-Quinolylmethyl | 1 | 57 | >300 |
| 95 | H | H | 3,4-Methylenedioxybenzyl | 1 | 67 | 215-218 |
| 96 | H | H | 2-Methoxybenzyl | 2 | 85 | 240-241 |
| 97 | H | H | 2-Methoxybenzyl | 3 | 59 | 186-188 |
| 98 | H | H | Cyclohexylmethyl | 1 | 79 | 249-251 |
| 99 | H | H | 3-Methoxybenzyl | 1 | 91 | 240-243 |
| 100 | H | H | 4-Methoxybenzyl | 1 | 90 | 209-211 |
| 101 | H | H | 2-Methoxyphenethyl | 1 | 67 | 220-221 |
| 102 | Methyl | H | 2-Methoxybenzyl | 1 | 79 | 252-255 |

Table 9

| Cpd. No. | $R^3$ | $R^{12}$ | $R^1$ | n | Yield (%) | M.p. (°C ) |
|---|---|---|---|---|---|---|
| 103 | Me | H | 2-(1-Methylindol-3-yl)-ethyl | 1 | 75 | 254-255 |
| 104 | * | H | 2-Methoxybenzyl | 1 | 90 | 180-185 |
| 105 | Me | 4-MeO | 2-Methoxybenzyl | 1 | 93 | 216-218 |
| 106 | Me | 4-Meo | 2-(1-Methylindol-3-yl)ethyl | 1 | 88 | 249-251 |
| 107 | Me | 4-Nitro | 2-Methoxybenzyl | 1 | 78 | >300 |
| 108 | Me | 4-MeO | H | 1 | 58 | 271-273.5 |
| 109 | Me | 4-MeO | Butyl | 1 | 96 | 195-197 |
| 110 | Me | 4-MeO | Benzyl | 1 | 78 | 194-194.5 |
| 111 | Me | 4-MeO | 2-Methylbenzyl | 1 | 87 | 239-240 |
| 112 | Me | 4-MeO | 2-Bromobenzyl | 1 | 86 | 231-233 |
| (Me: methyl; MeO: methoxy; *: (N-Methyl-N-phenyl)aminomethyl) | | | | | | |

Table 10

| Cpd. No. | $R^3$ | $R^{12}$ | $R^1$ | n | Yield (%) | M.p. (°C ) |
|---|---|---|---|---|---|---|
| 113 | Me | 4-MeO | 2-Nitrobenzyl | 1 | 58 | 190-191 |
| 114 | Me | 4-MeO | 2-Cyanobenzyl | 1 | 71 | 260-262 |
| 115 | Me | 4-MeO | 2-Methylthiobenzyl | 1 | 87 | 185-189 |
| 116 | Me | 4-MeO | 2-Methoxyphenethyl | 1 | 82 | 201.5-203 |
| 117 | Me | 4-MeO | 1-Naphthylmethyl | 1 | 83 | 209-210.5 |
| 118 | Me | 4-MeO | 2-Naphthylmethyl | 1 | 68 | 254-258 |
| 119 | Me | 2-MeO | 2-Methoxybenzyl | 1 | 67 | 216-217.5 |
| 120 | Me | 4-MeO | 2-Methoxybenzyl | 2 | 84 | 231-233 |
| 121 | Me | 4-MeO | 2-Methoxybenzyl | 3 | 86 | 190-191 |
| 122 | Me | 4-MeO | 3-Methoxybenzyl | 1 | 54 | 181-182 |

Table 11

| Cpd. No. | $R^3$ | $R^{12}$ | $R^1$ | n | Yield (%) | M.p. (°C ) |
|---|---|---|---|---|---|---|
| 123 | Me | 4-Methoxy | 4-Methoxybenzyl | 1 | 81 | 193-195 |
| 124 | Me | 4-Methoxy | 2,3-Dimethoxybenzyl | 1 | 59 | 192-914 |
| 125 | Me | 4-Methoxy | 2,5-Dimethoxybenzyl | 1 | 73 | 176-178 |
| 126 | Me | 4-Hydroxy | 2-Methoxybenzyl | 1 | 69 | 232-234 |
| 127 | Me | 4-(2-Methoxybenzyloxy) | 2-Methoxybenzyl | 1 | 82 | 123-124.5 |

Table 11 (continued)

| Cpd. No. | $R^3$ | $R^{12}$ | $R^1$ | n | Yield (%) | M.p. (°C ) |
|---|---|---|---|---|---|---|
| 128 | Me | 4-Ethoxy | 2-Methoxybenzyl | 1 | 66 | 227-228 |
| 129 | Me | 4-iso-Propoxy | 2-Methoxybenzyl | 1 | 80 | 220-223 |
| 130 | Me | 4-(2-Hydroxyethoxy) | 2-Methoxybenzyl | 1 | 67 | 198-200 |
| 131 | Me | 4-Methoxymethoxy | 2-Methoxybenzyl | 1 | 88 | 149-151 |
| 132 | Me | 4-Methoxy | 2-Hydroxybenzyl | 1 | 64 | 250-254 |
| 133 | Me | 4-Methoxy | 2-Ethoxybenzyl | 1 | 88 | 167-170 |
| 134 | Me | 4-Methoxy | 2-Benzyloxybenzyl | 1 | 62 | 200-202 |
| 135 | Me | 4-Methoxy | 2'-Cyanobiphenylmethyl | 1 | 84 | 214-215 |
| 136 | Me | 4-Methoxy | 2-Methoxymethoxybenzyl | 1 | 76 | 151-153 |
| 137 | Me | 4-Methoxy | 2-Benzimidazolmethyl | 1 | 54 | >300 |
| 138 | Me | 4-Methoxy | 2-Picolyl | 1 | 83 | 262-265 |
| 139 | Me | 4-Methoxyethoxy | 2-Methoxybenzyl | 1 | 86 | 192-193 |
| 140 | Me | 4-Methylthiomethoxy | 2-Methoxybenzyl | 1 | 82 | 164-168 |
| 141 | Me | 4-Carboxylmethoxy | 2-Methoxybenzyl | 1 | 65 | 212-215 |
| 142 | Me | 4-Benzyloxy | 2-Methoxybenzyl | 1 | 76 | 206-207 |
| 143 | Me | 4-Carbamoylmethoxy | 2-Methoxybenzyl | 1 | 76 | 243-248 |
| 144 | Me | 4-Methoxymethoxy | 2-Methylthiobenzyl | 1 | 79 | 142-144 |
| 145 | Me | 4-Methylthiomethoxy | 2-Methylthiobenzyl | 1 | 79 | 152-154 |
| 146 | Me | 4-n-Propoxy | 2-Methylthiobenzyl | 1 | 78 | 185-188 |
| 147 | Me | 4-Methoxyethoxymethoxy | 2-Methylthiobenzyl | 1 | 86 | 150-155 |
| 148 | Me | 4-(2-Oxopropoxy) | 2-Methylthiobenzyl | 1 | 78 | 285-290 |
| 149 | Me | 4-Methoxy | 2-Methylsulfinylbenzyl | 1 | 67 | 223-224 |
| 150 | Me | 4-Methoxy | 2-Methylsulfonylbenzyl | 1 | 96 | 142-145 |
| 151 | Me | 4-n-Butoxy | 2-Methylthiobenzyl | 1 | 72 | 178-180 |
| 152 | Me | 4-Methoxy | 2-Fluorobenzyl | 1 | 59 | 196-197 |
| 153 | Me | 4-Allyloxy | 2-Methylthiobenzyl | 1 | 81 | 198-200 |
| 154 | Me | 4-Methoxy | 2-tert-Butoxycarbonylbenzyl | 1 | 95 | 140-144 |
| 155 | Me | 4-Methoxy | 2-Carboxybenzyl | 1 | 86 | 289-291 |
| 156 | Me | 3,4-Methylenedioxy | 2-Methylthiobenzyll | 1 | 94 | 205-210 |
| 157 | Me | 3,4-Dimethoxy | 2-Methilthiobenzyl | 1 | 78 | 203-205 |
| 158 | Me | 4-Methoxy | 2,4-Dimethoxybenzyl | 1 | 82 | 203-204 |
| 159 | Me | 4-Butyryl | 2-Methylthiobenzyl | 1 | 54 | 197-199 |
| 160 | Me | 4-Etylthiomethyl | 2-Methylthiobenzyl | 1 | 43 | 286-289 |
| 161 | Me | 4-Methoxy | 2-Aminobenzyl | 1 | 48 | 209-212 |
| 162 | Me | 4-Methoxy | 2-Methylaminobenzyl | 1 | 58 | 204-206 |
| 163 | * | 4-Methoxymethoxy | 2-Methoxybenxyl | 1 | 91 | 220-225 |
| 164 | Me | 4-Hydroxyl | 2-Methylthiobenzyl | 1 | 72 | 244-246 |
| 165 | Me | 4-Phenyl | 2-Methylthiobenzyl | 1 | 53 | 270-273 |
| 166 | Me | 4-Methoxyethyl | 2-Methylthiobenzyl | 1 | 73 | 186-187 |

(Me: methyl, *: hydroxymethyl)

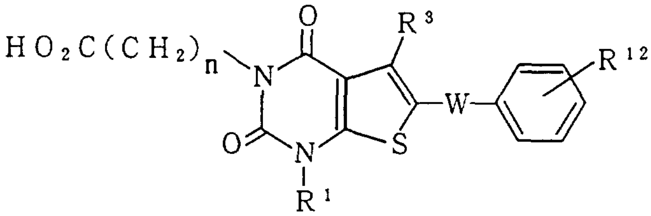

Table 12

| Cpd. No. | $R^3$ | W | $R^{12}$ | $R^1$ | n | Yield (%) | M.p. (°C) |
|---|---|---|---|---|---|---|---|
| 167 | Me | Sulfopiperazinyl | H | 2-Methoxybenzyl | 1 | 58 | 239-242 |
| 168 | Me | Carbonyl | H | 2-Methylthiobenzyl | 1 | 27 | 214-216 |
| 169 | Me | Carbonyl | 4-MeO | 2-Methylthiobenzyl | 1 | 58 | 202-206 |
| 170 | Me | Amide | 4-MeO | 2-Methylthiobenzyl | 1 | 83 | >300 |
| (Me: methyl) | | | | | | | |

Example 24.

Production of ethyl 2,4(1H,3H)-dioxo-1-(2-methylthiobenzyl)-6-(4-aminophenyl)-5-methylthieno[2,3-d]pyrimidine-3-acetate (Compound 171).

To a solution of the compound produced in Example 7 (Compound 36, 3.0 g, 6.48 mmoles) in ethanol (40 ml) were added iron powder (1.2 g) and concentrates hydrochloric acid (2.0 ml). The mixture was refluxed for 30 minutes. After cooling, the reaction mixture was filtered through cellite (10 g), the filtrate was concentrated to dryness. The residue was partitioned between ethyl acetate (100 ml) and a saturated aqueous solution of sodium bicarbonate (30 ml). The aqueous layer was extracted with ethyl acetate. The combined organic extracts were washed with an aqueous solution of sodium chloride, dried over $MgSO_4$ and evaporated in vacuo. The residue was recrystalized from ethyl acetate-isopropylether to give colorless prisms (2.08 g, 69%).

M.p. 172-173°C .

| Elemental analysis (%) for $C_{25}H_{25}N_3O_4S_2$: | | | |
|---|---|---|---|
| Calcd. | C 60.59, | H 5.08, | N 8.48 |
| Found | C 60.56, | H 4.93, | N 8.49. |

$^1$H-NMR (500MHz, $CDCl_3$) δ : 1.30 (3H, t, J = 7.0Hz), 2.49 (3H, s), 2.52 (3H, s), 3.80 (2H br s), 4.25 (2H, q, J=7.1Hz), 4.84 (2H, s), 5.33 (2H, s), 6.66(2H, d, J=8.3Hz), 7.02 (1H, d, J=7.7Hz), 7.12 (2H, d J=8.3Hz), 7.14 (1H, t, J=8.1Hz), 7.25 (1H, t, J=8.1Hz), 7.33 (1H, d, J=7.8Hz).

Example 25.

Production of ethyl 2,4(1H,3H)-dioxo-1-(2-methylthiobenzyl)-6-[4-(1-pyrrolyl)phenyl]-5-methylthieno[2,3-d]pyrimidine-3-acetate (Compound 172)

To a suspension of the compound produced in Example 24 (0.3 g, 0.65 mmoles) in acetic acid (5 ml) were added 2,5-dimethoxytetrahydrofurane (0.086 g) and sodium acetate (53 mg) and the mixture was refluxed for 30 minutes. The reaction mixture was concentrated and the residue was partitioned between ethy acetate (50 ml) and a saturated aqueous solution of ammonium chloride (30 ml). The aqueous layer was extracted with ethyl acetate (30 ml). The combined organic extracts were washed with an aqueous solution of sodium chloride, dried over $MgSO_4$ and evaporated in vacuo. The resulting residue was chromatographed on silica gel to give a white solid which was recrystallized from ether-ethyl acetate to give colorless prisms (0.28 g; 80%).

m.p. 156-158°C .

| Elemental analysis (%) for $C_{29}H_{27}N_3O_4S_2$: | | | |
|---|---|---|---|
| Calcd. | C 63.83, | H 4.99, | N 7.70; |
| Found | C 63.64, | H 4.99, | N 7.59. |

$^1$H-NMR (500MHz, $CDCl_3$) δ : 1.31 (3H, t, J = 7.0Hz), 2.53 (3H, s), 2.60 (3H, s), 4.26 (2H, q, J = 7.1Hz), 4.85 (2H, s), 5.35 (2H, s), 6.36 (2H, s), 7.03 (1H, d, J=7.7Hz), 7.09 (2H, d J=8.3Hz), 7.15 (1H, t, J=7.6Hz), 7.28 (1H, t, J=7.4Hz), 7.34 (1H, d, J=7.7Hz), 7.35-7.50 (4H, m) .

Example 26.

Production of 2,4(1H,3H)-dioxo-1-(2-methylthiobenzyl)-6-[4-(1-pyrrolyl)phenyl]-5-methylthieno[2,3-d]pyrimidine-3-acetic acid (Compound 173).

To a solution of the compound produced in Example 25 (0.10 g, 0.18 mmole) in a mixture of methanol (10 ml) and tetrahydrofuran (10 ml) was added 1N aqueous sodium hydroxide (2 ml) and the solution was stirred at room temperature for 3 hours. The reaction mixture was concentrated and the residue was partitioned between ethy acetate (50 ml) and 1N hydrochloric acid (3 ml). The aqueous layer was extracted with ethyl acetate (30 ml). The combined organic extracts were washed with an aqueous solution of sodium chloride, dried over $MgSO_4$ and evaporated in vacuo. The resulting residue was chomatographed on silica gel to give a white solid which was recrystallized from ether-ethyl acetate to give pale yellow crystals (0.05 g, 53%).

m.p. 192-197°C .

| Elemental analysis (%) for $C_{27}H_{23}N_3O_4S_2 \cdot 1.5H_2O$: | | | |
|---|---|---|---|
| Calcd. | C 59.54, | H 4.81, | N 7.72; |
| Found | C 56.58, | H 4.56, | N 7.73. |

$^1$H-NMR (500MHz, $CDCl_3$) δ : 2.52 (3H, s), 2.55 (3H, s), 4.59 (2H, s), 5.21 (2H, s), 6.28 (2H, s), 7.00 (2H, d, J = 7.6Hz), 7.14 (1H, t, J = 7.0Hz), 7.33 (1H, t, J = 7.98Hz), 7.40 (2H,s), 7.46 (2H, d, J=8.3Hz), 7.46 (2H, d, J=8.3Hz).

Example 27.

Production of ethyl 2,4(1H,3H)-dioxo-6-(4-butyrylphenyl)-5-methylthieno[2,3-d]pyrimidine-3-acetate (Compound 174).

To a solution of the compound produced in Example 4 (0.39 g, 1.10 mmoles) in nitrobenzene (10 ml) was added aluminum chloride (0.63 g, 4.75 mmoles) with ice-cooling under nitrogen stream. After stirring with ice-cooling for 30 minutes, to the solution was added dropwise butyryl chloride (0.21 ml, 2.20 mmoles). The mixture was stirred at 50°C for 3 days. The reaction mixture was poured into ice-water and the resulting mixture was partinioned between ethyl acetate and an aqueous solution of sodium chloride. The aqueous layer was extracted with ethyl acetate. The combined organic extracts were washed with an aqueous solution of sodium chloride, dried over $MgSO_4$ and evaporated in vacuo. The residue was chromatographed on silica gel to give white amorphous powders (0.05 g, 11%).

$^1$H-NMR (200MHz, $CDCl_3$) δ : 1.03 (3H, t, J = 7.2Hz), 1.32 (3H, t, J=7.2Hz), 1.77 (2H, sext, J=7.2Hz), 2.54 (3H, s), 2.98 (2H, t, J=7.2Hz), 4.27 (2H, q, J=7.2Hz), 4.78 (2H, s), 7.51 (2H, d, J=8.4Hz), 8.02 (2H, d, J=8.4Hz), 10.20 (1H, s).

FAB-MS m/z: 415.1 $(MH)^+$

Example 28.

Production of ethyl 2,4(1H,3H)-dioxo-6-(4-chloromethylphenyl)-5-methylthieno[2,3-d]pyrimidine-3-acetate (Compound 175).

To a solution of the compound produced in Example 4 (1.60 g, 4.65 mmoles) in nitromethane (40 ml) was added aluminum chloride (2.60 g, 19.5 mmoles) with ice-cooling under nitrogen stream. After stirring with ice-cooling for 30 minutes, to the solution was added dropwise methoxyacetyl chloride (0.64 ml, 7.00 mmoles). The mixture was stirred with ice-cooling for one hour and at 50°C for 24 hours. The reaction mixture was poured into ice-water and the resulting mixture was partitioned between ethyl acetate and an aqueous solution of sodium chloride. The aqueous layer was extracted with ethyl acetate. The combined organic extracts were washed with an aqueous solution of sodium chloride, dried over $MgSO_4$ and evaporated in vacuo. The residue was chromatographed on silica gel to give a white solid (0.74 g, 41%).

m.p. 177-179°C

$^1$H-NMR (200MHz, $CDCl_3$) δ : 1.31 (3H, t, J = 7.1Hz), 2.50 (3H, s), 4.26 (2H, q, J=7.1Hz), 4.63 (2H, s), 4.78 (2H, S), 7.39 (2H, d, J=8.1Hz), 7.47 (2H, d, J=8.1Hz), 9.92 (1H, s).

IR (KBr): 3242, 3004, 1738, 1659, 1560, 1526, 1493 $cm^{-1}$.

Example 29.

Production of ethyl 2,4(1H,3H)-dioxo-6-(4-ethylthiomethylpheny)-5-methylthieno[2,3-d]pyrimidine-3-acetate (Compound 176).

To a solution of the compound produced in Example 28 (0.30 g; 0.76 mmole) in dimethylformamide (10 ml) were added potassium iodide (0.13 g, 0.76 mmole), ethanthiol (1.00 ml, 11.9 mmoles) and ethyldiisopropylamine (0.16 ml, 0.92 mmole) with ice-cooling. The solution was stirred ice-cooling for 30 minutes and at room temperature for 4 hours. The reaction mixture was concentrated and the residue was partitioned between ethyl acetate and an aqueous solution of ammonium chloride. The aqueous layer was extracted with ethyl acetate. The combined organic extracts were washed with an aqueous solution of sodium chloride, dried over $MgSO_4$ and evaporated in vacuo. The resulting residue was chromatographed on silica gel to give an oily product (0.16 g, 50%).

$^1$H-NMR (200MHz, DMSO-$d_6$) $\delta$ : 1.21-1.29 (6H, m), 2.41-2.52 (5H, m), 3.73 (2H, s), 4.21 (2H, q, J=7.1Hz), 4.78 (2H, s), 7.29 (2H, q, J=8.5Hz), 7.35 (2H, d, J=8.5Hz), 11.18 (1H, s).

FAB-MS m/z: 419.1 $(MH)^+$

Example 30.

Production of ethyl 2,4(1H,3H)-dioxo-1-(2-aminobenzyl)-5-methyl-6-(4-methoxyphenyl)thieno[2,3-d]pyrimidine-3-acetate (Compound 177).

To a solution of the Compound 32 produced in Example 7 (0.60 g, 1.18 mmoles) in acetic acid (25 ml) was added iron powder (0.37 g, 5.89 mmoles). The mixture was stirred at 80°C for 2 hours. After cooling, the reaction mixture was concentrated and the residue was partitioned between ethyl acetate and a saturated aqueous solution of sodium bicarbonate. The aqueous layer was extracted with ethyl acetate. The combined organic extracts were washed with an aqueous solution of sodium chloride, dried over $MgSO_4$ and evaporated in vacuo. The residue was recrystalized from ethyl acetate-hexane to give colorless prisms (0.45 g, 80%).

m.p. 138-140°C.

| Elemental analysis (%) for $C_{25}H_{25}N_3O_5S{\cdot}0.2\ C_4H_8O_2$: | | | |
|---|---|---|---|
| Calcd. | C 62.32, | H 5.39, | N 8.45 |
| Found | C 62.36, | H 5.21, | N 8.25. |

$^1$H-NMR (200MHz, $CDCl_3$) $\delta$ : 1.31 (3H, t, J = 7.1Hz), 2.47 (3H, s), 3.85 (3H, s), 4.25 (2H, q, J=7.1Hz), 4.77 (2H, br s), 4.82 (2H, s), 5.16 (2H, s), 6.63-6.73 (2H, m), 6.94-7.36 (6H, m).

IR (KBr): 1754, 1705, 1636, 1562, 1528, 1502 $cm^{-1}$.

FAB-MS m/z: 480 $(MH^+)$

Example 31.

Production of ethyl 2,4(1H,3H)-dioxo-1-[2-(N-methylamino)benzyl]-5-methyl-6-(4-methoxyphenyl)thieno[2,3-d]pyrimidine-3-acetate (Compound 178).

To acetic anhydride (1.00 ml, 1.06 mmoles) was added dropwise formic acid (0.50 ml, 13.3 mmoles) with ice-cooling, the mixture was stirred at 55°C for 2 hours and then allowed to cool to -20°C, the compound produced in Example 30 (0.30 g, 0.62 mmole) was added thereto and the mixture was stirred at -20°C for 30 minutes. The reaction mixture was concentrated in vacuo to give white powders (0.45g, 80%). To a solution of these white powders (0.30 g, 0.59 mmole) in tetrahydrofuran (15 ml) was added dropwise dimethylsulfide-boric acid (0.25 ml, 2.50 mmoles) with ice-cooling. The mixture was stirred with ice-cooling for 30 minutes and then refluxed for 2 hours. The reaction mixture was cooled to 0°C and added methanol (1 ml) thereto and then the mixture was stirred at room temperature for one hour. To this solution was added a solution of hydrogen chloride in methanol (10N, 0.50 ml) and the mixture was refluxed for 30 minutes. The reaction mixture was concentrated and the residue was partitioned between ethyl acetate and a saturated aqueous solution of sodium bicarbonate. The aqueous layer was extracted with ethyl acetate. The combined organic extracts were washed with an aqueous solution of sodium chloride, dried over $MgSO_4$ and evaporated in vacuo. The residue was chromatographed on silica gel to give a curde product which was recrystalized from ethyl acetate-hexane to give white crystals (0.16 g, 55%).

m.p. 188-189°C

| Elemental analysis (%) for $C_{26}H_{27}N_3O_5S$: | | | |
|---|---|---|---|
| Calcd. | C 63.27, | H 5.51, | N 8.51; |
| Found | C 63.20, | H 5.69, | N 8.63. |

$^1$H-NMR (200MHz, $CDCl_3$) δ : 1.32 (3H, t, J = 7.1Hz), 2.46 (3H, s), 2.82 (3H, s), 3.85 (3H, s), 4.26 (2H, q, J=7.1Hz), 4.83 (2H, s), 5.15 (2H, s), 6.59-6.69 (2H, m), 6.95 (2H, q, J=8.8Hz), 7.21-7.36 (4H, m).
IR (KBr): 1742, 1696, 1647, 1607, 1570, 1535 $cm^{-1}$.
FAB-MS m/z: 494 ($MH^+$).

Example 32.

Production of ethyl 2,4(1H,3H)-dioxo-5-bromomethyl-1-(2-methoxybenzyl)-6-(4-methoxymethoxypheny)thieno[2,3-d]pyrimidine-3-acetate (Compound 179).

A mixture of the Compound 68 produced in Example 15 (0.69 g, 1.32 mmoles), N-bromosuccinimide (0.234 g, 1.32 mmoles), α ,α '-azobisisobutyronitrile (22 mg, 0.13 mmole) and carbontetrachloride (30 ml) was refluxed for 1.5 hours. After cooling, insoluble material was filtered off, the filtrate was diluted with chloroform and washed with an aqueous solution of sodium chloride, dried over $MgSO_4$ and evaporated in vacuo. The resulting residue was recrystalized from ethyl acetate-hexane to give white crystals (0.93 g, 82%).
m.p. 102-105°C

| Elemental analysis (%) for $C_{27}H_{27}N_2O_7SBr \cdot 0.2C_4H_8O_2 \cdot 0.3H_2O$: | | | |
|---|---|---|---|
| Calcd. | C 53.29, | H 4.69, | N 4.47; |
| Found | C 53.13, | H 4.43, | N 4.19. |

$^1$H-NMR (200MHz, $CDCl_3$) δ : 1.30 (3H, t, J = 7.2Hz), 3.50 (3H, s), 3.88 (3H, s), 4.25 (2H, q, J=7.2Hz), 4.79 (2H, s), 4.86 (2H, s), 5.22 (2H, s), 5.25 (2H, s), 6.88-7.50 (8H, m).
IR (KBr): 1746, 1707, 1665, 1607, 1528, 1479 $cm^{-1}$.

Example 33.

Production of ethyl 2,4(1H,3H)-dioxo-5-acetoxymethyl-1-(2-methoxybenzyl)-6-(4-methoxyphenyl)thieno[2,3-d]pyrimidine-3-acetate (Compound 180).

To a solution of the compound produced in Example 32 (0.30 g, 0.50 mmole) in dimethylformamide (10 ml) were added potassium carbonate (0.10 g, 0.74 mmole) and potassium acetate (0.10 g, 0.99 mmole) and the mixture was stirred at room temparature for 17 hours. The reaction mixture was concentrated and the residue was partitioned between ethyl acetate and a saturated aqueous solution of sodium chloride. The aqueous layer was extracted with ethyl acetate. The combined organic extracts were washed with an aqueous solution of sodium chloride, dried over $MgSO_4$ and evaporated in vacuo. The residue was chromatographed on silica gel to give white amorphous powders (0.24 g, 83%).
$^1$H-NMR (200MHz, $CDCl_3$) δ : : 1.30 (3H, t, J = 7.1Hz), 2.07 (3H, s), 3.49 (3H, s), 3.88 (3H, s), 4.24 (2H, q, J=7.1Hz), 4.83 (2H, s), 5.20 (2H, s), 5.24 (4H, s), 6.88-7.30 (8H, m).
IR (KBr): 1744, 1711, 1667, 1539, 1483 $cm^{-1}$.

Example 34.

Production of ethyl 2,4(1H,3H)-dioxo-6-(4-trifluoromethansulfonyloxyphenyl)-5-methyl-1-(2-methylthiobenzyl)thieno[2,3-d]pyrimidine-3-acetate (Compound 181).

To a solution of the Compound 63 produced in Example 13 (1.00 g, 2.01 mmoles) and ethyldiisopropylamine (0.42 ml, 2.41 mmoles) in dichloromethane (20 ml) was added trifluoromethansulonic anhydride (0.37 ml, 2.21 mmoles) with ice-cooling. The mixture was stirred with ice-cooling for 30 minutes and then at room temperature for 4 hours. The reaction mixture was partitioned between dichloromethane and an aqueous saturated solution of sodium chloride. The aqueous layer was extracted with dichloromethane. The combined organic extracts were washed with an aqueous

solution of sodium chloride, dried over $MgSO_4$ and evaporated in vacuo. The residue was chromatographed on silica gel to give white amorphous powders (1.05 g, 83%).

$^1$H-NMR (200MHz, $CDCl_3$) δ : 1.32 (3H, t, J = 7.2Hz), 2.53 (3H, s), 2.54 (3H, s), 4.26 (2H, q, J=7.2Hz), 4.85 (2H, s), 5.35 (2H, s), 7.01-7.44 (8H, m).

IR (KBr): 1750, 1711, 1669, 1562, 1528, 1475 $cm^{-1}$.

FAB-MS m/z: 628.9 ($MH^+$)

Example 35.

Production of ethyl 2,4(1H,3H)-dioxo-6-(4-biphenyl)-5-metyl-1-(2-methylthiobenzyl)thieno[2,3-d]pyrimidine-3-acetate (Compound 182).

Under argon stream, to a solution of the compound produced in Example 34 (0.40 g, 0.64 mmloe), phenylboric acid (0.08 g, 0.64 mmole) and 2M aqueous solution of sodium carbonate (1.60 ml, 3.20 mmoles) in 1,2-dimethoxyethane (20 ml) was added tetrakis(triphenylphosphine)palladium (0.11 g, 0.10 mmole) and the mixture was refluxed for 4 hours. The reaction mixture was diluted with ethyl acetate and an insoluble material was filtered off. The filtrate was partitioned between ethyl acetate and a saturated aqueous solution of sodium chloride. The aqueous layer was extracted with ethyl acetate. The combined organic extracts were washed with an aqueous solution of sodium chloride, dried over $MgSO_4$ and evaporated in vacuo. The residue was chromatographed on silica gel to give a curde product which was recrystalized from ethyl acetate-hexane to give white crystals (0.24 g, 67%).

m.p. 159-160°C

| Elemental analysis (%) for $C_{31}H_{28}N_2O_4S_2 \cdot 0.5C_4H_8O_2 \cdot 0.35CHCl_3$: | | | |
|---|---|---|---|
| Calcd. | C 62.34, | H 5.07, | N 4.35; |
| Found | C 62.34, | H 4.88, | N 4.17. |

$^1$H-NMR (200MHz, $CDCl_3$) δ : 1.32 (3H, t, J = 7.2Hz), 2.55 (3H, s), 2.59 (3H, s), 4.27 (2H, q, J=7.2Hz), 4.87 (2H, s), 5.37 (2H, s), 7.03-7.64 (13H, m).

IR (KBr) : 1742, 1709, 1661, 1533, 1475, 1446 $cm^{-1}$.

FAB-MS m/z: 557.2 ($MH^+$).

Example 36.

Production of ethyl 2,4(1H,3H)-dioxo-5-methyl-1-(2-methylthiobenzyl)-6-(4-methoxyethylphenyl)thieno[2,3-d]pyrimidine-3-acetate (Compound 183).

Under argon stream, to a solution of the compound produced in Reference Example 13 (0.40 g, 0.83 mmloe), 4-methoxyphenylboric acid (0.22 g, 1.24 mmoles) and 2M aqueous solution of sodium carbonate (2.10 ml, 4.20 mmoles) in 1,2-dimethoxyethane (20 ml) was added tetrakis(triphenylphosphine)palladium (0.14 g, 0.13 mmole) and the mixture was refluxed for 5 hours. The reaction mixture was diluted with ethyl acetate and an insoluble material was filtered off. The filtrate was partitioned between ethyl acetate and a saturated aqueous solution of sodium chloride. The aqueous layer was extracted with ethyl acetate. The combined organic extracts were washed with an aqueous solution of sodium chloride, dried over $MgSO_4$ and evaporated in vacuo. The residue was chromatographed on silica gel to give a curde product which was recrystalized from ethyl acetate-hexane to give white crystals (0.19 g, 43%).

m.p. 126-128°C

| Elemental analysis (%) for $C_{28}H_{30}N_2O_5S_2$: | | | |
|---|---|---|---|
| Calcd. | C 62.43, | H 5.61, | N 5.20; |
| Found | C 62.32, | H 5.39, | N 5.09. |

$^1$H-NMR (200MHz, $CDCl_3$) δ : 1.30 (3H, t, J = 7.2Hz), 2.51 (3H, s), 2.54 (3H, s), 2.89 (2H, t, J=6.9Hz), 3.35 (3H, s), 3.61 (2H, t, J=6.9Hz), 4.26 (2H, q, J=7.2Hz), 4.86 (2H, s), 5.34 (2H, s), 7.05-7.40 (8H, m).

IR (KBr): 1754, 1709, 1663, 1477 $cm^{-1}$.

FAB-MS m/z: 539.2 ($MH^+$).

Example 37.

Production of ethyl 2,4(1H,3H)-dioxo-6-thienyl-5-methyl-1-(2-methylthiobenzyl)thieno[2,3-d]pyrimidine-3-acetate (Compound 184).

3-Thiopeneboric acid (0.32 g, 1.24 mmoles) was used instead of 4-methoxyphenylboric acid and the same operations as mentioned in Example 36 were carried out by using the compound produced in Reference Example 13 (0.40 g, 0.83 mmoles), 2M aqueous solution of sodium carbonate (2.1 ml, 4.2 mmoles), dimethoxyethane (20 ml) and tetraxis(triphenylphoshine)palladium (0.20 g, 0.17 mmole) to give Compound 184 (yield: 45%).

m.p. 119-121°C

$^1$H-NMR (200MHz, $CDCl_3$) δ : 1.31 (3H, t, J = 7.1Hz), 2.54 (3H, s), 2.57 (3H, s), 4.25 (2H, q, J=7.1Hz), 4.85 (2H, s), 5.34 (2H, s), 7.00-7.35 (2H, m).

IR (KBr): 1709, 1665, 1541, 1477 $cm^{-1}$.

FAB-MS m/z: 494 ($MH^+$).

Example 38.

Production of ethyl 2,4(1H,3H)-dioxo-6-[4-(3-thienyl)phenyl]-5-methyl-1-(2-methylthiobenzyl)thieno[2,3-d]pyrimidine-3-acetate (Compound 185).

3-Thiopeneboric acid (0.14 g, 1.09 mmoles) was used instead of phenylboric acid and the same operations as mentioned in Example 35 were carried out by using the compound produced in Example 34 (0.33 g, 0.53 mmoles), 2M aqueous solution of sodium carbonate (1.3 ml, 2.6 mmoles), dimethoxyethane (15 ml) and tetraxis(triphenylphoshine)palladium (0.11 g, 0.10 mmole) to give Compound 185 (yield: 50%).

m.p. 166-168°C

| Elemental analysis (%) for $C_{29}H_{26}N_2O_4S_3 \cdot 0.5H_2O$: | | | |
|---|---|---|---|
| Calcd. | C 61.47, | H 4.79, | N 4.89; |
| Found | C 61.82, | H 4.81, | N 4.60. |

$^1$H-NMR (200MHz, $CDCl_3$) δ : 1.31 (3H, t, J = 7.1Hz), 2.54 (3H, s), 2.57 (3H, s), 4.26 (2H, q, J=7.1Hz), 4.86 (2H, s), 5.36 (2H, s), 7.01-7.64 (11H, m).

IR (KBr): 1736, 1707, 1665, 1475 $cm^{-1}$.

Example 39.

Production of ethyl 2,4(1H,3H)-dioxo-5-methyl-1-(2-methylthiobenzyl)-6-(4-propyonylaminophenyl)thieno[2,3-d]pyrimidine-3-acetate (Compound 180).

To a solution of the compound produced in Example 24 (0.30 g, 0.61 mmole) in dichloromethane (10 ml) was added triethylamine (0.10 g, 0.73 mmole) and then added N,N-dimethylaminopyridine (15 mg, 0.12 mmole) and propionyl lchloride (0.11 ml, 1.22 mmoles). The mixture was stirred at room temparature for one hour. The reaction mixture was partitioned between aq. sat. $NH_4Cl$ solution water and dichloromethane. The aqueous layer was extracted with dichloromethane. The combined organic extracts were washed with an aqueous solution of sodium chloride, dried over $MgSO_4$ and evaporated in vacuo. The residue was chromatographed on silica gel to give colorless crystals (0.25 g, 74%).

m.p. 218-219

| Elemental analysis (%) for $C_{26}H_{25}N_3O_5S_2 \cdot 0.5H_2O$: | | | |
|---|---|---|---|
| Calcd. | C 58.63, | H 4.92, | N 7.89; |
| Found | C 58.71, | H 4.90, | N 7.79. |

$^1$H-NMR (200MHz, $CDCl_3$) δ : 1.22-1.34 (6H, m), 2.41 (2H, q, J = 7.5Hz), 2.51 (3H, s), 2.53 (3H, s), 4.26 (2H, q, J=7.1Hz), 4.85 (2H, s), 5.32 (2H, s), 7.00-7.56 (8H, m).

IR (KBr): 1707, 1661, 1537, 1479 $cm^{-1}$.

Example 40.

Production of 2,4(1H,3H)-dioxo-6-thienyl-5-methyl-1-(2-methylthiobenzyl)thieno[2,3-d]pyrimidine-3-acetic acid (Compound 187).

The same operations as mentioned in Example 22 were carried out by using the compound produced in Example 37 (0.10 g, 0.21 mmole), 1N aqueous solution of sodium hydroxide (1.03 ml, 1.03 mmoles), tetrahydrofuran (3 ml) and ethanol (3 ml) to give Compound 187 (yield: 59%).

m.p. 255-257°C

| Elemental analysis (%) for $C_{21}H_{18}N_2O_4S_3$: | | | |
|---|---|---|---|
| Calcd. | C 55.00, | H 3.96, | N 6.11; |
| Found | C 54.78, | H 3.87, | N 6.14. |

$^1$H-NMR (200MHz, $CDCl_3$) $\delta$ : 2.53 (3H, s), 2.58 (3H, s), 4.64 (2H, s), 6.98 (1H, d, J = 7.5Hz), 7.15 (1H, t, J = 7.5HZ), 7.23-7.45 (3H, m), 7.67-7.71 (2H, m).

IR (KBr): 1696, 1659, 1635, 1477 $cm^{-1}$.

Example 41.

A tablets was prepared by a conventional method using 100 mg of the compound 131 produced in Example 23, 165 mg of lactose, 25 mg of corn starch, 4 mg of polyvinyl alcohol and 1 mg of magnesium stearate.

Example 42.

The compound 131 (5 g) produced in Example 23 was dissolved in a distilled water for injection to make the total volume 100 ml. The solution was subjected to an aseptic filtration using a membrane filter of 0.22 micrometer (manufactured by Sumitomo Electric, Japan or by Saltorius, Germany). Each 2 ml of the filtrate was placed in a washed and sterilized vial and dried by freezing by a conventional method to prepare a freeze-dried injection of 100 mg/vial.

Example 43.

A tablet was prepared by a conventional method using 100 mg of the compound 115 produced in Example 23, 165 mg of lactose, 25 mg of corn starch, 4 mg of polyvinyl alcohol and 1 mg of magnesium stearate.

Example 44.

The compound 115 (5 g) produced in Example 23 was dissolved in a distilled water for injection to make the total volume 100 ml. The solution was subjected to an aseptic filtration using a membrane filter of 0.22 micrometer (manufactured by Sumitomo Electric, Japan or by Saltorius, Germany). Each 2 ml of the filtrate was placed in a washed and sterilized vial and dried by freezing by a conventional method to prepare a freeze-dried injection of 100 mg/vial.

Example 45.

A tablet was prepared by a conventional method using 100 mg of the compound 144 produced in Example 23, 165 mg of lactose, 25 mg of corn starch, 4 mg of polyvinyl alcohol and 1 mg of magnesium stearate.

Example 46.

The compound 144 (5 g) produced in Example 23 was dissolved in a distilled water for injection to make the total volume 100 ml. The solution was subjected to an aseptic filtration using a membrane filter of 0.22 micrometer (manufactured by Sumitomo Electric, Japan or by Saltorius, Germany). Each 2 ml of the filtrate was placed in a washed and sterilized vial and dried by freezing to prepare a freeze-dried injection of 100 mg/vial.

Example 47.

A tablet was prepared by a conventional method using 100 mg of the compound 145 produced in Example

23, 165 mg of lactose, 25 mg of corn starch, 4 mg of polyvinyl alcohol and 1 mg of magnesium stearate.

Example 48.

The compound 145 (5 g) produced in Example 23 was dissolved in a distilled water for injection to make the total volume 100 ml. The solution was subjected to an aseptic filtration using a membrane filter of 0.22 micrometer (manufactured by Sumitomo Electric, Japan or by Saltorius, Germany). Each 2 ml of the filtrate was placed in a washed and sterilized vial and dried by freezing by a conventional method to prepare a freeze-dried injection of 100 mg/vial.

Test Example 1.

Endothelin Receptor Assay:

A pig ventricular muscle membrane fraction for an endothelin-A receptor assay and a bovine cerebral membrane fraction for an endothelin-B receptor assay were diluted with an assay buffer [comprising 20 mM of Tris-HCl, 2 mM of EGTA (ethylene glycol bis(2-aminoethyl ether)tetraacetic acid), 5 mM of magnesium acetate, 0.1% of BSA (bovine serum albumin), 0.03% of $NaN_3$, 0.5 mM of PMSF (phenylmethylsulfonyl fluoride), 20 micrograms/ml of leupeptin, 4 micrograms/ml of E-64 (manufactured by the Peptide Institute, Japan) and 1 microgram/ml of pepstatin; pH 7.2] to 12 µg/ml and 180 µg/ml, respectively.

To each 100 microliter of them was added 5 nM [$^{125}I$] endothelin-1 (2 microliters). A solution (3 microliters) of the sample was added and the mixture was incubated at 25°C for 60 minutes. In order to measure the maximum binding ($B_0$) and the nonspecific bonding (NSB), a lot to which dimethyl sulfoxide (3 microliters) or a solution (3 microliters) of 10-5M of endothelin in dimethyl sulfoxide was added was incubated as well. An assay buffer supplemented with 0.05% CHAPS (3-[(3-cholamidopropyl)dimethylammonio]-1-propanesulfonate, 1.5 ml) was added thereto and the mixture was filtered through a GF/F glass fiber filter (a trade name of Whatman, England) and further washed with 1.5 ml of the same buffer. The radioactivity of the filter was counted by a γ -counter and a calculation was conducted in accordance with the following formula (1) whereby a percent maximum binding (hereinafter, referred to as PMB) was determined. In addition, the concentration wherein the PMB was 50% ($IC_{50}$) was calculated as well. The $IC_{50}$ values for the compounds of the present invention are shown in the following Table 13.

$$PMB = [(B - NSB)/(B_0 - NSB)] \times 100 \qquad (1)$$

Table 13

| | $IC_{50}$ (micromoles) | |
|---|---|---|
| Compound Number | Endothelin-A Receptor (pigs) | Endothelin-B Receptor (bovine) |
| Compd. No. 102 | 1.3 | 7.5 |
| Compd. No. 99 | 12 | 39 |

Test Example 2.

Endothelin Assay Test:

Endothelin (ET) receptors were prepared as follows. An insect cell (Sf9) membrane fraction wherein human endothelin-A (ETA) was expressed and that wherein endothelin-B (ETB) was expressed were diluted with an assay buffer [comprising 20 mM of Tris-HCl, 2 mM of EGTA (ethylene glycol bis(2-aminoethyl ether)tetraacetic acid), 5 mM of magnesium acetate, 0.1% of BSA (bovine serum albumin), 0.03% of $NaN_3$, 0.5 mM of PMSF (phenylmethylsulfonyl fluoride), 20 micrograms/ml of leupeptin, 4 micrograms/ml of E-64 (manufactured by the Peptide Institute, Japan) and 1 microgram/ml of pepstatin; pH 7.2] to make 1.4 micrograms/ml and 0.7 microgram/ml for the insect cell (Sf 9) membrane fraction wherein a human ETA receptor was expressed and for that wherein a human ETB receptor was expressed, respectively. Each 100 microliters of them were taken out and 2 microliters of 5nM [$^{125}I$]endothelin-1 was added thereto. A dimethyl sulfoxide solution (3 microliters) of the sample was added thereto and the mixture was incubated at 25°C for 60 minutes. Further, in order to measure the maximum binding ($B_0$) and the nonspecific binding (NSB), a lot to which 3 ml of dimethyl sulfoxide or 3 ml solution of 10-5M of endothelin-1 in dimethyl sulfoxide was added was incubated as well. An assay buffer suspended with 0.05% CHAPS (3-[(3-cholamidopropyl)di-methylammonio]-1-propanesulfonate, 1.5 ml) was added and the mixture was filtered through a GF/F glass fiber filter (trade name of Whatman, England) followed by washing with 1.5 ml of the same buffer.

The radioactivity of the filter was counted by a $\gamma$ -counter and determined PMB by the same way described in Test Example 1. The value wherein PMB = 50% was calculated and expressed as an $IC_{50}$. The $IC_{50}$ values of the compounds of the present invention are shown in the following Table 14.

Table 14

| | $IC_{50}$ (micromoles) | |
|---|---|---|
| Compound Number | Endothelin-A Receptor (human) | Endothelin-B Receptor (human) |
| 105 | 1.9 | 12 |
| 111 | 1.8 | 14 |
| 115 | 0.71 | 6.8 |
| 125 | 2.9 | 26 |
| 130 | 1.2 | 12 |
| 131 | 0.30 | 2.4 |
| 140 | 0.24 | 15 |
| 143 | 2.5 | 33 |
| 144 | 0.085 | 0.92 |
| 145 | 0.066 | 0.66 |
| 146 | 0.25 | 2.6 |
| 148 | 0.40 | 7.1 |
| 156 | 2.6 | 16 |
| 173 | 2.1 | 2.5 |

(Effect of the Invention)

The pharmaceutical composition for antagonizing endothelin containing the thienopyrimidine derivatives of the present invention can be advantageously used for preventing or treating acute renal insufficiency, myocardial infarction, liver insufficiency, angina pectoris, cerebral infarction, sub-arachnoid heamorrhage (SAH), hypertension, renal insufficiency, asthma, variant form of angina, Raynaud's syndrome, pulmonary hypertension, surgery shock, chronic cardiac insufficiency, cardiac hypertrophy, arteriosclerosis, migraine and the like, furthermore, for treating or preventing a hypofunction of an organ caused by its surgery or transplant insufficient microcirculation, still furthermore, for preventing restenosis after PTCA.

## Claims

**1.** A compound of the formula:

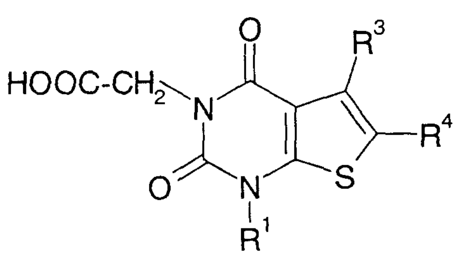

wherein

$R^1$ is benzyl which may be substituted by substituents selected from the group consisting of $C_{1-6}$ alkyl, $C_{1-6}$ alkoxy and $C_{1-6}$ alkylthio,
$R^3$ is hydrogen or $C_{1-6}$ alkyl,
$R^4$ is phenyl which may be substituted by $C_{1-6}$ alkoxy or $C_{1-6}$ alkylthio,

or a salt thereof.

2. A compound according to claim 1, wherein $R^4$ is phenyl which may be substituted by $C_{1-6}$ alkoxy.

3. A compound selected from the group consisting of

2,4(1H,3H)-dioxo-5-methyl-6-(4-methoxymethoxyphenyl)-1-(2-methoxybenzyl)thieno[2,3-d]pyrimidine-3-acetic acid,
2,4(1H,3H)-dioxo-5-methyl-6-(4-methoxyphenyl)-1-(2-methylthiobenzyl)thieno[2,3-d]pyrimidine-3-acetic acid,
2,4(1H,3H)-dioxo-5-methyl-6-(4-methoxymethoxyphenyl)-1-(2-methylthiobenzyl)thieno[2,3-d]pyrimidine-3-acetic acid,
2,4(1H,3H)-dioxo-5-methyl-6-(4-methylthiomethoxyphenyl)-1-(2-methylthiobenzyl)thieno[2,3-d]pyrimidine-3-acetic acid,
2,4(1H,3H)-dioxo-5-methyl-6-(4-methylthiomethoxyphenyl)-1-(2-methoxybenzyl)thieno[2,3-d]pyrimidine-3-acetic acid,
2,4(1H,3H)-dioxo-5-methyl-1-(2-methylthiobenzyl)-6-(4-propoxyphenyl)thieno[2,3-d]pyrimidine-3-acetic acid, and
2,4(1H,3H)-dioxo-5-methyl-1-(2-methylthiobenzyl)-6-[4-(2-oxopropoxy)phenyl]thieno[2,3-d]pyrimidine-3-acetic acid.

4. A pharmaceutical composition which comprises a compound according to any one of claims 1 to 3 and a pharmaceutically acceptable carrier, excipient or diluent.

5. Use of a compound according to any one of claims 1 to 3 and a pharmaceutically acceptable carrier, excipient or diluent for producing a pharmaceutical composition for antagonizing endothelin activity in a mammal suffering from an endothelin-derived disorder.

6. Use according to claim 5, wherein the composition is prophylactic or therapeutic composition for acute renal insufficiency, myocardial infarction and/or liver insufficiency.

7. Use according to claim 5, wherein the composition is prophylactic or therapeutic composition for hypofunction of an organ caused by its surgery or transplant.

8. Use according to claim 7, wherein the organ is liver.

**Patentansprüche**

1. Verbindung der Formel

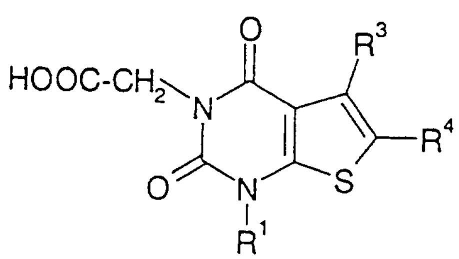

wobei

$R^1$ Benzyl ist, das mit Substituenten substituiert sein kann, die aus der Gruppe ausgewählt sind, die aus $C_{1-6}$-Alkyl, $C_{1-6}$-Alkoxy und $C_{1-6}$-Alkylthio besteht;

$R^3$ Wasserstoff oder $C_{1-6}$-Alkyl ist;

$R^4$ Phenyl ist, das mit $C_{1-6}$-Alkoxy oder $C_{1-6}$-Alkylthio substituiert sein kann;

oder ein Salz davon.

**2.** Verbindung gemäß Anspruch 1, wobei $R^4$ Phenyl ist, das mit $C_{1-6}$-Alkoxy substituiert sein kann.

**3.** Verbindung, die aus der Gruppe ausgewählt ist, die aus folgenden besteht:

2,4(1H,3H)-Dioxo-5-methyl-6-(4-methoxymethoxyphenyl)-1-(2-methoxybenzyl)thieno[2,3-d]pyrimidin-3-essigsäure;
2,4(1H,3H)-Dioxo-5-methyl-6-(4-methoxyphenyl)-1-(2-methylthiobenzyl)-thieno[2,3-d]pyrimidin-3-essigsäure;
2,4(1H,3H)-Dioxo-5-methyl-6-(4-methoxymethoxyphenyl)-1-(2-methylthiobenzyl)thieno[2,3-d]pyrimidin-3-essigsäure;
2,4(1H,3H)-Dioxo-5-methyl-6-(4-methylthiomethoxyphenyl)-1-(2-methylthiobenzyl)thieno[2,3-d]pyrimidin-3-essigsäure;
2,4(1H,3H)-Dioxo-5-methyl-6-(4-methylthiomethoxyphenyl)-1-(2-methoxybenzyl)thieno[2,3-d]pyrimidin-3-essigsäure;
2,4(1H,3H)-Dioxo-5-methyl-1-(2-methylthiobenzyl)-6-(4-propoxyphenyl)-thieno[2,3-d]pyrimidin-3-essigsäure; und
2,4(1H,3H)-Dioxo-5-methyl-1-(2-methylthiobenzyl)-6-[4-(2-oxopropoxy)-phenyl]thieno[2,3-d]pyrimidin-3-essigsäure.

**4.** Pharmazeutische Zusammensetzung, die eine Verbindung gemäß einem der Ansprüche 1 bis 3 sowie einen pharmazeutisch annehmbaren Träger, Arzneimittelhilfsstoff oder ein pharmazeutisch annehmbares Verdünnungsmittel umfasst.

**5.** Verwendung einer Verbindung gemäß einem der Ansprüche 1 bis 3 sowie eines pharmazeutisch annehmbaren Trägers, Arzneimittelhilfsstoffs oder Verdünnungsmittels zur Herstellung einer pharmazeutischen Zusammensetzung zum Antagonisieren von Endothelin-Aktivität bei einem Säuger, der unter einer durch Endothelin verursachten Störung leidet.

**6.** Verwendung gemäß Anspruch 5, wobei die Zusammensetzung eine prophylaktische oder therapeutische Zusammensetzung gegen akute Niereninsuffizienz, Myokardinfarkt und/oder Leberinsuffizienz ist,

**7.** Verwendung gemäß Anspruch 5, wobei die Zusammensetzung eine prophylaktische oder therapeutische Zusammensetzung gegen Unterfunktion eines Organs ist, die durch dessen Operation oder Transplantation verursacht ist.

**8.** Verwendung gemäß Anspruch 7, wobei das Organ die Leber ist.

### Revendications

**1.** Composé de formule :

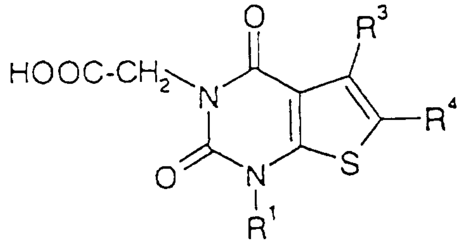

dans laquelle

$R^1$ représente un groupe benzyle, qui peut porter des substituants choisis dans l'ensemble constitué par les groupes alkyle en $C_{1-6}$, alcoxy en $C_{1-6}$ et alkylthio en $C_{1-6}$,
$R^3$ représente un atome d'hydrogène ou un groupe alkyle en $C_{1-6}$,
et $R^4$ représente un groupe phényle qui peut porter un substituant alcoxy en $C_{1-6}$ ou alkylthio en $C_{1-6}$,

ou sel d'un tel composé.

2. Composé conforme à la revendication 1, dans lequel $R^4$ représente un groupe phényle qui peut porter un substituant alcoxy en $C_{1-6}$.

3. Composé choisi dans l'ensemble que constituent :

l'acide 2,4(1H,3H)-dioxo-5-méthyl-6-(4-méthoxyméthoxyphényl)-1-(2-méthoxybenzyl)-thiéno[2,3-d]pyrimidine-3-acétique,
l'acide 2,4(1H,3H)-dioxo-5-méthyl-6-(4-méthoxyphényl)-1-(2-méthylthiobenzyl)-thiéno[2,3-d]pyrimidine-3-acétique,
l'acide 2,4(1H,3H)-dioxo-5-méthyl-6-(4-méthoxyméthoxyphényl)-1-(2-méthylthiobenzyl)-thiéno[2,3-d]pyrimidine-3-acétique,
l'acide 2,4(1H,3H)-dioxo-5-méthyl-6-(4-méthylthiométhoxyphényl)-1-(2-méthylthiobenzyl)-thiéno[2,3-d]pyrimidine-3-acétique,
l'acide 2,4(1H,3H)-dioxo-5-méthyl-6-(4-méthylthiométhoxyphényl)-1-(2-méthoxybenzyl)-thiéno[2,3-d]pyrimidine-3-acétique,
l'acide 2,4(1H,3H)-dioxo-5-méthyl-1-(2-méthylthiobenzyl)-6-(4-propoxyphényl)-thiéno[2,3-d]pyrimidine-3-acétique, et
l'acide 2,4(1H,3H)-dioxo-5-méthyl-1-(2-méthylthiobenzyl)-6-[4-(2-oxopropoxy)phényl]-thiéno[2,3-d]pyrimidine-3-acétique.

4. Composition pharmaceutique contenant un composé conforme à l'une des revendications 1 à 3 et un véhicule, excipient ou diluant admissible en pharmacie.

5. Emploi d'un composé conforme à l'une des revendications 1 à 3 et d'un véhicule, excipient ou diluant admissible en pharmacie pour la préparation d'une composition pharmaceutique destiné à s'opposer à l'activité de l'endothéline chez un mammifère souffrant d'un trouble lié à l'endothéline.

6. Emploi conforme à la revendication 5, ladite composition étant une composition prophylactique ou thérapeutique contre l'insuffisance rénale aiguë, l'infarctus du myocarde et/ou l'insuffisance hépatique.

7. Emploi conforme à la revendication 5, ladite composition étant une composition prophylactique ou thérapeutique contre l'hypofonctionnement d'un organe causé par un acte chirurgical effectué sur cet organe ou une transplantation de cet organe.

8. Emploi conforme à la revendication 7, ledit organe étant le foie.